# EUROPÄISCHE PATENTSCHRIFT

(11) **EP 1 410 793 B1**
(45) Veröffentlichungstag und Bekanntmachung des Hinweises auf die Patenterteilung: **16.11.2005**
(21) Anmeldenummer: 03405225.8
(22) Anmeldetag: 03.04.2003
(51) Int. Cl.: A61K 9/20

(54) **Darreichungsform von Ibuprofen-Natrium**
Delivery form of sodium ibuprofen
Composition pharmaceutique d'ibuprofène

(30) Priorität: 14.10.2002 CH 17032002
(43) Veröffentlichungstag der Anmeldung: 21.04.2004
(73) Patentinhaber: Bayer Consumer Care AG, 4052 Basel (CH)
(72) Erfinder: Gruber, Peter, Dr., 79249 Merzhausen (DE); Reher, Markus, Dr., 79410 Badenweiler (DE)
(74) Vertreter: Hoechst, Bruno Werner

(56) Entgegenhaltungen:
- EP-A- 0 418 043
- WO-A-00/06125
- US-A- 5 104 648
- US-A- 5 424 075

## Beschreibung

Die Erfindung betrifft eine nicht brausende Tablettenformulierung zur oralen Verabreichung von Ibuprofen-Natrium und ein Verfahren zu deren Herstellung.

Ibuprofen, d.h. 2-(4-Isobutylphenyl)propionsäure, ist ein bekanntes Medikament mit analgetischen, antiphlogistischen und antipyretischen Eigenschaften, das insbesondere zur Behandlung von entzündlichen Erkrankungen und Schmerzen, wie rheumatische Erkrankungen, Kopfschmerzen, Migräne, Zahnschmerzen, Rückenschmerzen, Muskelschmerzen, postoperative Schmerzen und dergleichen eingesetzt wird. Die therapeutisch wirksame Form ist das S(+)-Ibuprofen, während das R(-)-Enantiomer praktisch unwirksam ist, aber im Körper teilweise in die wirksame S(+)-Form umgewandelt wird. Obwohl in den letzten Jahren einige Präparate in den Handel gelangt sind, die das Ibuprofen in der S(+)-Form enthalten, wird Ibuprofen weiterhin meist in racemischer Form eingesetzt.

Ein wesentlicher Punkt insbesondere bei der Schmerzbehandlung ist die Erzielung einer rasch einsetzenden Wirkung. Um dies zu erreichen, muss der Wirkstoff rasch freigesetzt und resorbiert werden, was im Falle fester Darreichungsformen zudem voraussetzt, dass diese im Gastrointestinaltrakt rasch zerfallen. Andererseits müssen feste Darreichungsformen klein genug sein, dass sie noch problemlos geschluckt werden können.

Dabei stellen sich jedoch im Falle von Ibuprofen eine Reihe von Formlierungsproblemen. Einerseits enthalten Einheitsdosen von Ibuprofen-Formulierungen typischerweise Wirkstoffmengen entsprechend 200 mg, 400 mg, 600 mg oder 800 mg racemischem Ibuprofen, d.h. der Wirkstoffanteil einer Tablette muss hoch sein, damit diese noch schluckbar ist. Andererseits müssen die Formulierungen geeignete Hilfsstoffe in ausreichender Menge enthalten, damit die Formulierungen auf üblichen Tablettiermaschinen gut verpressbar sind, nicht an den Tablettierwerkzeugen kleben und rasch zerfallende Tabletten mit ausreichender Härte ergeben. Die Erzielung einer rasch einsetzenden Wirkung wird zudem dadurch erschwert, dass Ibuprofen in sauren Medien, insbesondere in Magensäure, nur sehr schlecht löslich ist, womit die Auflösung und Resorption des Wirkstoffes erheblich verzögert wird.

Wirkstoffe mit niedrigem Schmelzbereich, wie Ibuprofen, können bei der Tablettierung infolge von Sintervorgängen und durch Kleben an den Stempeln und Matrizen der Tablettenpresse zu Produktionsproblemen führen. Das Kleben kann zwar durch Zusatz grosser Mengen an Antiklebemitteln behoben werden. Dadurch werden die Endmischungen jedoch hydrophobisiert und somit die Wirkstofffreisetzung verlangsamt. Um dies zu vermeiden und gut fliessfähige, tablettierbare Pulvergemische zu erhalten wurde in WO-A-93/23026 vorgeschlagen, 100 Gewichtsteile von Ibuprofen oder anderen 2-Arylpropionsäuren mit 50-500 Gewichtsteilen an Calciumverbindungen wie Calciumhydrogenphosphat, Calciumcarbonat oder Calciumhydroxid trocken zu mischen und zusammen mit üblichen Hilfs- und/oder Trägerstoffen zu Tabletten zu verpressen.

In EP-A1-0 478 838 wurde demgegenüber zur Verbesserung der Tablettierbarkeit vorgeschlagen, Ibuprofen ganz oder teilweise in das Calciumsalz umzuwandeln und das Produkt unter Einsatz üblicher Zusatz- und Trägerstoffe, wie mikrokristalline Cellulose, Zerfallsbeschleuniger, Gleit- und Schmiermittel, zu granulieren und zu tablettieren. Der Wirkstoff kann gemäss EP-A1-0 478 838 neben dem CalciumSalz auch einen Anteil an Ibuprofen oder dessen Ammonium-, Natrium- oder Kaliumsalz enthalten, wobei die Ammonium- und Alkalisalze je nach Anteil die Löslichkeit verbessern aber zugleich die Hygroskopizität und die Klebrigkeit wieder erhöhen. Das zur Erhöhung des Schmelzbereichs und zur Verbesserung der Tablettierbarkeit verwendete Calciumsalz ist jedoch schlecht löslich, wodurch die Auflösung und Resorption verzögert wird.

In JP-A-63 198 620 wurde zur Vermeidung von Nebenwirkungen vorgeschlagen, Ibuprofen zusammen mit einem Antacidum (Aluminiumglycinat, Natriumhydrogencarbonat, Aluminiumlactat und/oder ein Copräzipitat von Magnesiumhydroxid und Kaliumsulfat) und/oder einem Coatingmittel für die Schleimhaut zu verwenden.

In US-A-4 834 966 wurde die Verwendung von Natriumbicarbonat in einer wasserlöslichen Zusammensetzung beschrieben, die ein angenehm schmeckendes Getränk ergeben soll und die 33-46 Gew.-% Ibuprofen, 34-51 Gew.-% L-Arginin und 9-29 Gew.-% Natriumbicarbonat enthält.

Aus US-A-5 262 179 sind ferner nicht brausende, wasserlösliche Sachet-Formulierungen bekannt, die ein Kalium-, Natrium-, Arginin- oder Lysinsalz von Ibuprofen und, um den Geschmack des Ibuprofens in der wässrigen Lösung zu maskieren, ein Bicarbonat, Hydrogenphosphat oder dreibasisches Citrat eines Alkalimetalls enthalten. Die offenbarten Formulierungen werden durch blosses Mischen der Komponenten erhalten und enthalten jeweils gegen 50 Gew.-% oder mehr an weiteren Hilfsstoffen, insbesondere Dextrose, und nur etwa 20 Gew.-% oder weniger an Ibuprofensalz.

In EP-A1-0 607 467 wurden schnell freisetzende S(+)-Ibuprofen-Pellets beschrieben, die 0,1-10,0 Gew.-% an basischen anorganischen Salzen, wie Natriumcarbonat, Dinatriumhydrogenphosphat oder Kaliumcarbonat, oder verdünnter Alkalilauge enthalten. Letztere bewirken während des Pelletiervorgangs ein schwaches Anlösen des S(+)-Ibuprofens, worauf dieses leicht klebrig wird, so dass sich die Verwendung von zusätzlichen Bindemitteln erübrigt. Die Pellets können mit üblichen Überzügen, insbesondere Schutzcoatings, magensaftresistente Coatings oder retardierende Coatings, versehen werden. Die gecoateten Pellets können, falls gewünscht, mittels herkömmlicher Verfahren zu Tabletten verpresst werden, die auf 400 mg S(+)-Ibuprofen 73-410 mg, vorzugsweise 240-260 mg Tablettierhilfsmittel, wie mikrokristalline Cellulose, Stärke, Crosscarmellose-Natrium, Magensiumstearat etc., enthalten. Die in EP-A1-0 607 467 angegebene schnelle Wirkstofffreisetzung gemäss USP XXII in Phosphatpuffer (pH 7,2) sagt jedoch nichts über die Schnelligkeit der Resorption einer solchen Arzneiform unter in vivo-Bedinungen aus, da die Löslichkeit von Ibuprofen und dessen Enantiomeren extrem pH-abhängig ist. Während Ibuprofen bei pH 7,2 durch Salzbildung rasch in Lösung übergeht, ist es in saurem Medium nur wenig löslich. Im Magen herrschen jedoch saure pH-Verhältnisse und selbst im oberen Darmabschnitt werden in der Regel pH-Werte von 7 nicht erreicht. Dies führt dazu, dass Ibuprofen erst allmählich in tieferen Darmabschnitten durch Salzbildung in Lösung geht und somit ein rasches Anfluten eines Wirkstoffspiegels nicht möglich ist.

Aus WO-A-97/30699 ist ferner eine nicht brausende Tablette bekannt, die ein Ibuprofen-Medikament in einer Menge von mindestens 35 Gew.-%, ein Trägermaterial, umfassend eine kompressible Füllstoffkomponente in Kombination mit einer Sprengmittelkomponente, und ferner im Trägermaterial ein Alkalimetallcarbonat oder -bicarbonat in ausreichender Menge enthält, dass die Darreichungsform eine Härte im Bereich von 6,5-15 Kp und eine Zerfallszeit von weniger als 10 Minuten aufweist, mit der Massgabe, dass das Ibuprofen-Medikament nicht ein Calciumsalz von Ibuprofen in Verbindung mit einem Alkalimetallsalz von Ibuprofen enthält. Gemäss der Offenbarung von WO-A-97/30699 sollen Alkalimetallcarbonate und -bicarbonate, die normalerweise nicht als kompressible Materialien verwendet werden, geeignet sein, die Verpressbarkeit von Zusammensetzungen zu erhöhen, die einen kompressiblen Füllstoff in Kombination mit einem Sprengmittel enthalten.

Das Ibuprofen-Medikament in der Dosierungsform gemäss WO-A-97/30699 kann Ibuprofen, eines seiner Enantiomere oder ein Salz oder Hydrat davon sein. Besonders vorteilhaft soll die Dosierungsform zur Formulierung der schlecht verpressbaren Alkalimetallsalze und vor allem des Natriumsalzes sein, das als flockig, weich, klebrig, besonders schlecht verpressbar und auch schlecht ganulierbar beschrieben wird. Als Füllstoff wird vorzugsweise ein Cellulosederivat, insbesondere mikrokristalline Cellulose, und als Sprengmittel vorzugsweise Crosscarmellose-Natrium oder Natriumstärkeglykolat verwendet. Die beschriebene Formulierung kann weitere Hilfsstoffe, wie Verdünnungmittel, Schmiermittel und Fliessmittel, enthalten und einen Zucker- oder Filmüberzug aufweisen. Die offenbarten Formulierungsbeispiele enthalten meist etwa 50 Gew.-% an Ibuprofen-Natrium-Dihydrat, und etwa 50 Gew.-% an Hilfsstoffen, nämlich mikrokristalline Cellulose und gegebenenfalls Lactose als Füllstoffe, vernetztes Polyvinylpyrrolidon oder Crosscarmellose-Natrium als Sprengmittel, Magnesiumstearat, Stearinsäure oder Pflanzenöl als Schmiermittel, Alkalimetallcarbonat oder -bicarbonat und gegebenenfalls Talk oder Siliziumdioxid als Fliessmittel.

Die auf dem Markt erhältlichen Ibuprofen-Präparate (z.B. NUROFEN, Boots) enthalten den Wirkstoff zumeist in Form der Säure, die jedoch in sauren Medien und somit im Magen und im oberen Darmbereich schlecht löslich ist. Es sind viele Versuche unternommen worden, die Resorption und damit den Aufbau ausreichender Blutspiegel zu beschleunigen, um eine rascher einsetzende schmerzlindernde Wirkung zu erzielen. Diese Entwicklungen haben zu einer Reihe von am Markt erhältlichen Tablettenformulierungen geführt, die statt des im pH-Bereich der Magensäure schwer löslichen Ibuprofens Ibuprofenlysinat (z.B. DOLORMIN, Woelm Pharma GmbH&Co., Bad Honnef, Deutschland) oder Ibuprofenarginat (z.B. DOLO-SPEDIFEN 200, Inpharzam AG, Cadempino, Schweiz) enthalten. Die Aminosäuren Lysin und Arginin sind aber sehr teuer und verteuern damit die entsprechenden Formulierungen. Zudem bedingt die Verwendung dieser Salze den Einsatz deutlich höherer Wirkstoffmengen und erhöht damit das Tablettengewicht, da z.B. die einer Dosis von 200 mg Ibuprofen äquivalente Menge im Falle von Ibuprofenlysinat 342 mg und im Falle von Ibuprofenarginat 369 mg beträgt. Beispielsweise haben die einer Ibuprofen-Dosis von 200 mg bzw. 400 mg entsprechenden DOLORMIN-Tabletten ein Tablettengewicht von 400 mg bzw. 800 mg; im Falle der 400 mg-Dosis handelt es sich um eine oblonge Tablette mit den bereits beachtlichen Abmessungen von 19,3 mm Länge, 8,6 mm Breite und 6,6 mm Höhe, die von vielen Patienten nicht mehr problemlos geschluckt werden kann. Die einer Dosis von 200 mg Ibuprofen entsprechende Tablette DOLO-SPEDIFEN 200 hat ein Tablettengewicht von 610 mg, weshalb eine entsprechende Tablette für die doppelte Dosis nicht mehr realisierbar ist. Die Verwendung von Ibuprofenlysinat oder Ibuprofenarginat ist daher nur im Falle niedriger Dosierungen ein brauchbare, aber teure Alternative zur Verwendung von Ibuprofen.

Am Markt sind ferner Weichgelatinekapseln (SPALT LIQUA, Whitehall-Much, Münster, Deutschland) erhältlich, die zwecks rascher Resorption 200 mg gelöstes Ibuprofen unter Zusatz von einer geringen Menge Kalilauge enthalten. Für die Herstellung der Kapseln wird jedoch ein teures, spezielles Equipment benötigt, über das nur wenige Spezialfirmen verfügen. Zudem kann die Dosierung mangels Teilbarkeit der Kapsel nicht individuell angepasst werden. Entsprechende Kapseln mit 400 mg Ibuprofen sind bisher nicht erhältlich und wären zudem sehr gross und wenig schluckfreundlich.

Die Ammonium- und Alkalimetallsalze von Ibuprofen sind als klebrige, hygroskopische und schlecht verpressbare Substanzen bekannt. Insbesondere gilt das Natriumsalz aufgrund seiner wachsartigen Struktur als ausserordentlich schlecht verpressbar und auch als schlecht ganulierbar (K.D. Rainsford, "Ibuprofen: A critical bibliographic review", Verlag: Taylor & Francis, 1999, ISBN 0-7484-0694-8, Seite 75). Dies ist auch der Grund dafür, dass bisher keine Ibuprofen-Natrium enthaltenden Tabletten auf dem Markt erhältlich sind.

Der Erfindung liegt daher die Aufgabe zugrunde, eine produktionstechnisch herstellbare Tablettenformulierung bereitzustellen, die eine rasche Freisetzung und Resorption des Wirkstoffes gestattet und die dennoch vergleichsweise kleine Tablettengrössen ermöglicht.

Die Aufgabe wird gelöst durch eine nicht-brausende Tablette zur oralen Verabreichung von Ibuprofen-Natrium, umfassend einen Tablettenkern und gewünschtenfalls einen Zucker- oder Filmüberzug auf dem Tablettenkern, worin der Tablettenkern, bezogen auf das Gewicht des Tablettenkerns, aus 50 bis 100 Gew.-% Ibuprofen-Natrium-Hydrat und 50 bis 0 Gew.-% Hilfsstoffkomponente besteht und kein Schmiermittel und kein Sprengmittel enthält, wobei das Ibuprofen-Natrium-Hydrat einen Wassergehalt von 8 bis 16 Gew.-%, bezogen auf das Hydrat und gemessen als Trocknungsverlust bei 105°C, aufweist.

Überraschenderweise wurde nämlich gefunden, dass die Tablettierbarkeit von Ibuprofen-Natrium stark von dessen Wassergehalt abhängt und dass es entgegen der gängigen Lehrmeinung durchaus möglich ist, Tabletten mit ausreichender Härte und kurzen Zerfallszeiten herzustellen, die vergleichsweise wenig oder gar keinen Hilfsstoff enthalten, wenn ein Ibuprofen-Natrium-Hydrat mit einem Wassergehalt von 8 bis 16 Gew.-%, vorzugsweise 11 bis 16 Gew.-%, verwendet und der Wassergehalt genau kontrolliert wird. Aufgrund der im Stand der Technik beschriebenen, besonders schlechten Kompressionseigenschaften und der wachsartigen Struktur würde der Fachmann normalerweise gar nicht versuchen, eine weitgehend hilfsstofffreie Tablette herzustellen, sondern vergleichsweise hohe Mengen an kompressiblen Füllstoffen und Sprengmitteln zusetzen, um dennoch brauchbare Kompressions- und Zerfallseigenschaften zu erhalten. Es war daher völlig überraschend, dass sich bei geeignetem Wassergehalt sogar Tabletten aus reinem Ibuprofen-Natrium-Hydrat herstellen liessen.

In graphischer Darstellung zeigen
- Fig. 1: die Härte und die Zerfallszeit einer erfindungsgemässen Tablette in Abhängigkeit der bei der Tablettierung angewendeten Presskraft,
- Fig. 2: die Auflösungsprofile erfindungsgemässer Tabletten in 0,1 M Salzsäure (pH 1,2) nach der Paddle-Methode bei 50 UpM,
- Fig. 3: die Auflösungsprofile erfindungsgemässer Filmtabletten in 0,1 M Salzsäure (pH 1,2) nach der Paddle-Methode bei 100 UpM im Vergleich zu einer Dolormin-Filmtablette und einer Nurofen-Filmtablette,
- Fig. 4: die Auflösungsprofile erfindungsgemässer Filmtabletten in McIlvain-Puffer (pH 3,5) nach der Paddle-Methode bei 100 UpM im Vergleich zu einer Dolormin-Filmtablette und einer Nurofen-Filmtablette, und
- Fig. 5: die Auflösungsprofile erfindungsgemässer Filmtabletten in USP-Puffer (pH 7,2) nach der Paddle-Methode bei 50 UpM im Vergleich zu einer Dolormin-Filmtablette und einer Nurofen-Filmtablette.

Ibuprofen-Natrium kann grundsätzlich wasserfrei sein oder als Mono- oder Dihydrat oder als Gemisch dieser Formen vorliegen. Die wasserfreie Form und das Monohydrat sind hygroskopisch und nehmen unter Bildung des Dihydrats Wasser auf. Beispielsweise nimmt wasserfreies Ibuprofen-Natrium schon bei einer relativen Luftfeuchtigkeit von 25% r.F. spontan bis zu etwa 13,6 Gew.-% an Wasser auf. Bei Verwendung des Monohydrats würde man daher eine hygroskopische Tablette erhalten und ein sehr dichtes Packmittel benötigen; andernfalls würde die Tablette stark Wasser aufnehmen, erweichen und Deckelneigung zeigen. Im Falle von Filmtabletten wäre zudem die Ausdehnung der Tablette durch Wasseraufnahme so gross, dass der Filmüberzug gesprengt würde.

Das Dihydrat ist hingegen praktisch nicht mehr hygroskopisch und nimmt bei Raumtemperatur bis zu einer relativen Luftfeuchtigkeit von 90% r.F. weniger als 0,5 Gew.-% weiteres Wasser auf. Beispielsweise nahm Ibuprofen-Natrium-Hydrat mit einem Wassergehalt im Bereich von 13-14 Gew.-% selbst bei offener Lagerung über 6 Monate bei 40°C und 75% r.F. kein weiteres Wasser auf. Es ist durchaus üblich, dass das Ibuprofen-Natrium-Dihydrat von den Herstellern mit einem dem Dihydrat nicht entsprechenden Wassergehalt angeliefert wird, da bei der Trocknung das Kristallwasser schon bei 40-50°C leicht abgegeben wird und die Substanz beim Trocknen leicht in das Monohydrat übergeht. Diese Tatsache illustriert die Wichtigkeit einer genauen Kontrolle des Wassergehalts, und sie mag ebenfalls erklären, warum die Abhängigkeit der Tablettierbarkeit vom Wassergehalt im Stand der Technik nicht erkannt worden ist.

Liegt der Wassergehalt des Ibuprofen-Natrium-Hydrats unter 11 Gew.-%, wird es immer schwieriger, ausreichend harte Tabletten ohne Neigung zum Deckeln (capping) herzustellen und das Kleben an den Tablettierwerkzeugen zu vermeiden. Beträgt der Wassergehalt etwa 8 bis 11 Gew.-%, lassen sich diese Nachteile weitgehend durch Zusatz geeigneter Hilfsstoffe komponsieren. Beträgt der Wassergehalt des Ibuprofen-Natrium-Hydrats hingegen etwa 5 Gew.-% oder weniger, lässt sich eine Tablette mit wenig Hilfsstoff praktisch nicht mehr herstellen.

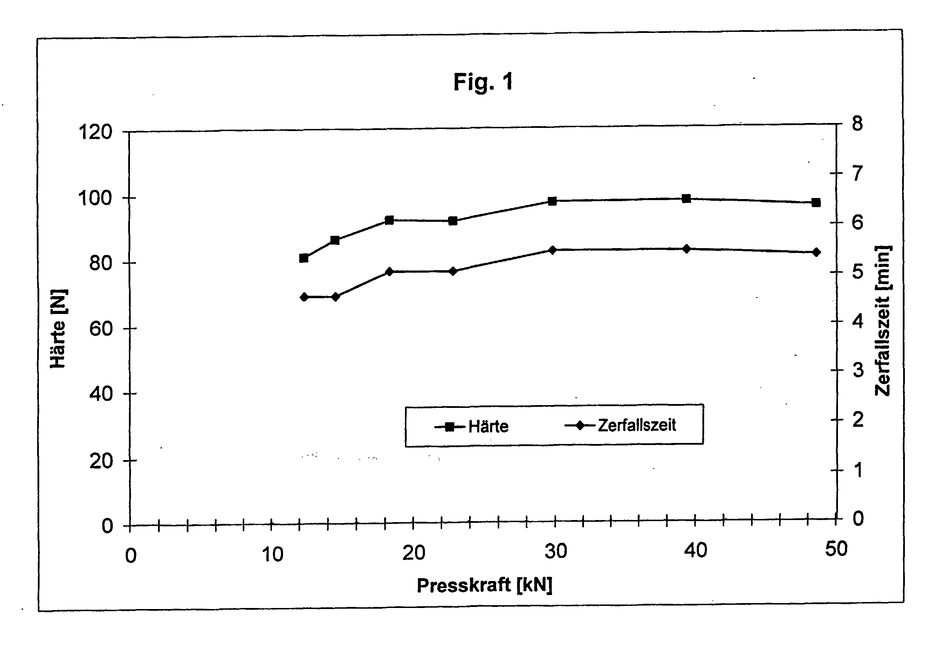

Überraschenderweise wurde ferner gefunden, dass die Härte und die Zerfallszeiten der erfindungsgemässen Tabletten trotz Fehlens eines Sprengmittels nahezu unabhängig von dem bei der Tablettierung angewendeten Pressdruck sind. Fig. 1 illustriert in graphischer Darstellung die mittels eines Schleuniger-Härtetesters gemessenen Härte und die in Wasser bei 37°C gemessenen Zerfallszeiten in Abhängigkeit von der Presskraft für eine erfindunsgemässe Tablette, bestehend aus 512,5 mg Ibuprofen-Natrium-Hydrat (Wassergehalt zwischen 13 und 14 Gew.-%), 50 mg Polyvinylpyrrolidon K25 und 99,5 mg Natriumhydrogencarbonat. Wie ersichtlich ist, führt eine Erhöhung der Presskraft von 20 auf 50 kN nur zu einer unbedeutenden Erhöhung der Härte und der Zerfallszeit. Aufgrund dieses überraschenden Befundes kann die Gefahr, dass durch Anwendung eines zu hohen Pressdruckes Tabletten mit zu hoher Härte und beeinträchtigten Zerfalls- und Freisetzungseigenschaften entstehen könnten, praktisch ausgeschlossen werden, was die Produktion der erfindungsgemässen Tabletten zusätzlich erleichtert.

Weiterhin wurde überraschenderweise gefunden, dass die erfindungsgemässen Tabletten ohne Zusatz eines inneren Schmiermittels wie Magnesiumsterat, Calciumstearat, Stearinsäure, Fett-Triglyceride und dergleichen hergestellt werden können. Bekanntlich müssen Tablettiermischungen üblicherweise Schmiermittel zugesetzt werden, damit es nicht zum Kleben an den Tablettierwerkzeugen kommt und die Reibung beim Ausstossen der Tabletten nicht zu hoch wird. Ohne Einsatz eines Schmiermittels kommt es normalerweise zu erheblichen Tablettierstörungen, die zur Folge haben, dass die Tablettierpressen abgestellt werden müssen und die Tabletten unbrauchbar sind, da sie beim Ausstossen aus der Maschine verletzt werden. Es war daher völlig überraschend, dass zur Herstellung der erfindungsgemässen Tabletten auf Schmiermittel verzichtet und auf üblichen Produktions-Tablettierpressen Millionen von Tabletten ohne jeglichen Zusatz eines inneren Schmiermittels abgepresst werden konnten. Tatsächlich wurde festgestellt, dass die Gefahr, dass die Endmischung an den Stempeloberflächen klebt, durch Zusatz klassischer Schmiermittel wie Magnesiumstearat sogar erhöht wird. Zudem sind die üblichen Schmiermittel hydrophob und würden die Verpressbarkeit und die Zerfallseigenschaften verschlechtern. Die erfindungsgemässen Tablettenformulierungen enthalten daher zweckmässigerweise keine signifikanten Mengen (d.h. weniger als 0,1 Gew.-%) an Schmiermitteln im Tablettenkern und sind mit Vorteil völlig frei von inneren Schmiermitteln.

Ferner hat sich gezeigt, dass es infolge des Verzichts auf innere Schmiermittel auch nicht mehr erforderlich ist, der Tablettiermischung ein Sprengmittel zuzusetzen. Damit kann der Anteil an Hilfsstoffen weiter verringert oder auf Hilfsstoffe sogar völlig verzichtet werden. Die Wasserlöslichkeit des Ibuprofen-Natrium-Hydrats ist tatsächlich so gross, dass es nicht gelingt, den Zerfall der Tablette durch Zusatz üblicher Sprengmittel oder Kombinationen von Füllstoffen wie mikrokristalline Cellulose mit Sprengmitteln weiter zu verbessern. Die erfindungsgemässen Tablettenformulierungen enthalten daher zweckmässigerweise keine signifikanten Mengen (d.h. weniger als 0,1 Gew.-%) an Sprengmitteln oder Füllstoffen mit Sprengmitteleigenschaften, wie vernetzte Polyvinylpyrrolidone, Magnesium-Aluminium-Silikate, mikrokristalline Cellulose, Stärken, Natriumcarboxymethylcellulose-Stärken etc., und sie sind mit Vorteil völlig frei von solchen Stoffen.

Die Zerfallszeiten der erfindungsgemässen Tabletten liegen in der Regel deutlich unter 10 Minuten, typischerweise im Bereich von etwa 2 bis 7 Minuten. Aufgrund der hohen Wasserlöslichkeit des Ibuprofen-Natrium-Hydrats und des Verzichts auf innere Schmiermittel führen die erfindungsgemässen Tabletten zu einer besonders raschen Freisetzung und Resorption des Wirkstoffes und damit zu einem raschen Anstieg des Blutspiegels und der Wirkortkonzentration. Zudem wurde gefunden, dass die erfindungsgemässen Tabletten, insbesondere wenn sie eine basische Komponente enthalten, in saurem Medium zu deutlich übersättigten Lösungen führen können, wodurch eine rasche Resorption zusätzlich begünstigt wird. Die vorliegende Erfindung ermöglicht daher im Vergleich zu bekannten Ibuprofen-Medikamenten sowohl eine raschere Erreichung wirksamer Blutspiegel und Wirkortkonzentrationen und damit eine beschleunigt einsetzende analgetische Wirkung als auch ein rascheres Erreichen der maximalen Blutspiegel und Wirkortkonzentrationen. Durch zahlreiche in vivo-Studien ist belegt, dass der maximale Blutspiegel mit herkömmlichen Ibuprofen-Formulierungen erst etwa 1,5 Stunden nach Verabreichung erreicht wird. Demgegenüber wurden mit erfindungsgemässen Tabletten ohne Sprengmittel bereits nach etwa 35 Minuten maximale Blutspiegel erreicht. Die erfindungsgemässen Tabletten gestatten somit eine besonders rasche Schmerzbekämpfung und sie verringern die Gefahr, dass der Patient infolge zu langsamem Einsetzen der analgetischen Wirkung eine weitere Tablette einnimmt.

Der Verzicht auf Schmiermittel und Sprengmittel und die Reduktion bzw. der Verzicht auf weitere Hilfsstoffe in den erfindungsgemässen Tablettenformulierungen ermöglichen eine deutliche Verringerung der Tablettengewichte und Tablettengrössen. Da die zu 200 mg Ibuprofen äquivalente Menge an Ibuprofen-Natrium-Dihydrat nur 256 mg beträgt ist der Gewichtsunterschied zum unlöslichen Ibuprofen nicht allzu gross, während gegenüber dem löslichen Ibuprofenlysinat und dem löslichen Ibuprofenarginat auch hinsichtlich des Wirkstoffes eine deutliche Gewichtseinsparung erzielt wird. Die erfindungsgemässen Tabletten sind folglich sowohl rasch resorbierbar als auch vergleichsweise klein.

Da die Herstellung der Tabletten in an sich bekannter Weise mit Standard-Tablettenpressen erfolgen kann, der Anteil an Hilfsstoffen gering gehalten werden kann und die Wirkstoffkosten im Vergleich zum Lysinat und Arginat gering sind, lassen sich die erfindungsgemässen Tabletten zudem besonders wirtschaftlich herstellen.

Der Ausdruck "Tablettenkern" bezeichnet im Rahmen der vorliegenden Erfindung eine Tablette ohne Zucker- oder Filmüberzug.

Der Ausdruck "Ibuprofen-Natrium-Hydrat" umfasst im Rahmen der vorliegenden Erfindung sowohl das Natriumsalz von racemischem Ibuprofen als auch die Natriumsalze der Enantiomeren S(+)-Ibuprofen und R(-)-Ibuprofen und von Gemischen dieser Enantiomeren. Bevorzugt verwendbar sind S(+)-Ibuprofen-Natrium-Hydrat und insbesondere racemisches Ibuprofen-Natrium-Hydrat. Der Wassergehalt des Hydrats beträgt zweckmässigerweise etwa 8 bis 16 Gew.-%, vorzugsweise etwa 11 bis 16 Gew.-%, bezogen auf das Gewicht des Hydrates; besonders bevorzugt ist ein Wassergehalt von etwa 12,5 bis 15 Gew.-%, insbesondere etwa 13 bis 14 Gew.-%. Aufgrund des erfindungemäss vorhandenen Wassergehaltes liegt das Hydrat meist überwiegend oder vollständig in Form des Dihydrates vor. Während sich geringe Anteile an Monohydrat kaum als störend erwiesen haben, tritt mit steigendem Monohydratanteil eine zunehmende Verschlechterung der Tablettierbarkeit ein, die bis zu einem gewissen Grad durch Hilfsstoffe kompensiert werden muss.

Im Rahmen der vorliegenden Erfindung wurde der Wassergehalt des Ibuprofen-Natrium-Hydrats jeweils als Trocknungsverlust bei 105°C bestimmt, da das Kristallwasser bei dieser Temperatur vollständig abgegeben wird.

Der Anteil an Ibuprofen-Natrium-Hydrat in den erfindungsgemässen Tablettenformulierungen kann, bezogen auf das Gewicht des Tablettenkerns, zweckmässigerweise etwa 50 bis 100 Gew.-%, vorzugsweise etwa 60 bis 100 Gew.-% und besonders bevorzugt etwa 70 bis 100 Gew.-% betragen. Dementsprechend beträgt der Anteil an Hilfsstoff im Tablettenkern zweckmässigerweise etwa 50 bis 0 Gew.-%, vorzugsweise etwa 40 bis 0 Gew.-% und besonders bevorzugt etwa 30 bis 0 Gew.-%.

Gemäss einer bevorzugten Ausführungsform kann der Tablettenkern im wesentlichen aus Ibuprofen-Natrium-Hydrat bestehen und im wesentlichen frei von Hilfsstoffen sein, d.h. vorzugsweise weniger als 0,1 Gew.-% oder besonders bevorzugt gar keinen Hilfsstoff enthalten. In dieser Ausführungsform sollte der Wassergehalt des Ibuprofen-Natrium-Hydrats vorzugsweise etwa 11 bis 16 Gew.-% betragen, wobei ein Wassergehalt von etwa 12,5 bis 15 Gew.-%, insbesondere etwa 13 bis 14 Gew.-% besonders bevorzugt sind. Zudem sollte die Tablette eine Tablettenhärte (gemessen mittels eines Schleuniger-Härtetesters) von vorzugsweise mindestens etwa 30 N, besonders bevorzugt mindestens etwa 40 N, aufweisen.

In der Regel ist es jedoch bevorzugt, im Tablettenkern einen geringen Anteil von mindestens etwa 0,1 Gew.-% an Hilfsstoff einzusetzen, der zweckmässigerweise im Gemisch mit dem Ibuprofen-Natrium-Hydrat vorliegt. Im Falle von Hilfsstoff enthaltenden Tablettenkernen kann daher der Anteil an Ibuprofen-Natrium-Hydrat, bezogen auf das Gewicht des Tablettenkerns, zweckmässigerweise etwa 50 bis 99,9 Gew.-%, vorzugsweise etwa 60 bis 99,9 Gew.-% und besonders bevorzugt etwa 70 bis 99,9 Gew.-% betragen. Der Hilfsstoffanteil im Tablettenkern beträgt dementsprechend zweckmässigerweise etwa 50 bis 0,1 Gew.-%, vorzugsweise etwa 40 bis 0,1 Gew.-% und besonders bevorzugt etwa 30 bis 0,1 Gew.-%.

Die im Tablettenkern verwendbaren Hilfsstoffe können grundsätzlich wasserlösliche oder in Wasser schlecht lösliche oder unlösliche Stoffe sein. Beispielsweise kann es gelegentlich erwünscht sein, in der Tablettiermischung ein unlösliches Bindemittel wie Siliziumdioxid zu verwenden. In der Regel ist es jedoch bevorzugt, im Tablettenkern überwiegend oder ausschliesslich wasserlösliche Hilfsstoffe zu verwenden. Als "wasserlöslich" werden im Rahmen der vorliegenden Erfindung Stoffe bezeichnet, die in Wasser bei 25°C in einer Konzentration von mindestens etwa 1 Gew.-% löslich sind.

Der Anteil an Hilfsstoffen (die vorzugsweise wasserlöslich sein können) kann, bezogen auf das Gewicht des Tablettenkerns, vorzugsweise etwa 7 bis 40 Gew.-%, besonders bevorzugt etwa 15 bis 30 Gew.-%, insbesondere etwa 20 bis 25 Gew.-%, betragen kann. Der Wirkstoffanteil im Tablettenkern kann daher vorzugsweise etwa 60 bis 93 Gew.-%, besonders bevorzugt etwa 70 bis 85 Gew.-%, insbesondere etwa 75 bis 80 Gew.-%, betragen.

Als Hilfsstoffkomponente im Tablettenkern eignen sich vorzugsweise Füllstoffe und/oder basische Hilfsstoffe. Ferner kann der Tablettenkern gewünschtenfalls eine geringe Menge an Tensid enthalten.

Bevorzugt verwendbare basische Hilfsstoffe sind solche Stoffe, die in einer Konzentration von 1 Gew.-% in Wasser bei 25°C eine wässrige Lösung oder Suspension mit einem pH-Wert von mindestens 7,5 ergeben. Beispiele bevorzugt verwendbarer basischer Hilfsstoffe sind basische Alkalimetallsalze, Erdalkalimetallsalze und Ammoniumsalze, beispielsweise in Form der Carbonate, Hydrogencarbonate, Phosphate, Hydrogenphosphate, Oxide, Hydroxide, Citrate, Tartrate, Acetate oder Propionate, insbesondere basische Natriumsalze, Kaliumsalze und Ammoniumsalze, wie Natriumhydrogencarbonat, Kaliumhydrogencarbonat, Natriumcarbonat, Kaliumcarbonat, Ammoniumcarbonat, Trinatriumcitrat, Dinatriumtartrat, Dikaliumtartrat, Magnesiumoxid, Calciumoxid, Magnesiumhydroxid, Calciumhydroxid, Magnesiumcarbonat, Calciumcarbonat, Dinatriumhydrogenphosphat, Dikaliumhydrogenphosphat, Trinatriumphosphat, Trikaliumphosphat, Tricalciumphosphat, Natriumacetat, Kaliumacetat, Natriumpropionat etc., basische Aminosäuren, wie Lysin und Arginin, und dergleichen. In der Regel sind wasserlösliche basische Hilfsstoffe wie Natriumhydrogencarbonat, Kaliumhydrogencarbont, Natriumcarbonat, Kaliumcarbonat, Trinatriumcitrat und Trinatriumphosphat bevorzugt. Besonders bevorzugt verwendbar sind Natriumhydrogencarbonat, Kaliumhydrogencarbonat oder ein Gemisch der beiden, insbesondere Natriumhydrogencarbonat.

Die basischen Hilfsstoffe begünstigen die Bildung eines schwach basischen Mikromilieus auf der Tablettenoberfläche und wirken damit vermutlich einer raschen Ausfällung des Ibuprofens im Magen entgegen. Der Anteil an basischem Hilfsstoff im Tablettenkern kann, falls vorhanden, bezogen auf das Gewicht des Tablettenkerns vorzugsweise etwa 5 bis 30 Gew.-%, insbesondere etwa 6 bis 25 Gew.-% betragen. Typischerweise werden meist etwa 8 bis 20 Gew.-%, insbesondere etwa 13 bis 17 Gew.-% an basischem Hilfsstoff verwendet. Ist der basische Hilfsstoff ein Hydrogencarbonat, wie Natrium- oder Kaliumhydrogencarbonat, kann der Anteil, bezogen auf das Ibuprofen-Natrium-Hydrat, vorzugsweise auch weniger als 1 Moläquivalent, beispielsweise etwa 0,2 bis 0,8 Moläquivalent betragen.

Als Füllstoffe im Tablettenkern eignen sich generell die Verpressbarkeit verbessernde Hilfsstoffe. Bevorzugt sind jedoch im allgemeinen neutrale bis schwach saure, die Verpressbarkeit verbessernde Füllstoffe, vorzugsweise solche, die keine Pufferwirkung ergeben. Im Rahmen der vorliegenden Erfindung umfasst der Ausdruck "neutraler bis schwach saurer Füllstoff" insbesondere Füllstoffe, die in einer Konzentration von 1 Gew.-% in Wasser bei 25°C eine wässrige Lösung oder Suspension mit einem pH-Wert zwischen 4 und 7,5 ergeben. Vorzugsweise werden wasserlösliche Füllstoffe verwendet. Beispiele bevorzugt verwendbarer Füllstoffe sind Zucker wie Saccharose, Glucose, Fructose und Lactose, Hexosen wie Mannit, Xylit, Maltit und Sorbit, hydrolysierte oder enzymatisch gespaltene Stärke wie Maltodextrin, Cyclodextrine wie β- und γ-Cyclodextrin, nicht vernetztes (wasserlösliches) Polyvinylpyrrolidon, Polyvinylalkohole, Polyethylenglykole, Polypropylenglykole, Alkalimetall-, Erdalkalimetall- und Ammoniumsalze organischer oder anorganischer Säuren, insbesondere Natrium-, Kalium-, Magnesium- und Calciumsalze, wie Natriumchlorid, Kaliumchlorid, Magnesiumchlorid, Natriumsulfat, Kaliumsulfat, Magnesiumsulfat, Trimagnesiumdicitrat, Tricalciumdicitrat, Calciumlactat, Calciumgluconat, Calciumhydrogenphosphat und dergleichen. Besonders bevorzugte Füllstoffe sind Hexosen wie Sorbit und Mannit, nicht vernetztes Polyvinylpyrrolidon, Maltodextrin und Natriumchlorid, insbesondere wasserlösliches, nicht vernetztes Polyvinylpyrrolidon, das offenbar zusätzlich geeignet ist, die Ausfällung des Ibuprofens im Magen zu verzögern. Als wasserlösliche, nicht vernetzte Polyvinylpyrrolidone eignen sich beispielsweise Povidone K25-K90 (BASF, Deutschland) wie Povidone K25 und Povidone K29-32.

Der Anteil an Füllstoff im Tablettenkern kann, falls vorhanden, bezogen auf das Gewicht des Tablettenkerns vorzugsweise etwa 1 bis 25 Gew.-%, insbesondere etwa 3 bis 20 Gew.-% und typischerweise etwa 5 bis 15 Gew.-% betragen.

Die erfindungsgemässe Tablettenformulierung kann Füllstoffe oder basische Hilfsstoffe oder beides enthalten. Enthält der Tablettenkern sowohl Füllstoff als auch basischen Hilfstoff können die optimalen Mengen gelegentlich etwas niedriger sein als die oben genannten Mengen. Die Gesamtmenge an Füllstoff und basischem Hilfstoffe beträgt zudem zweckmässigerweise höchstens etwa 50 Gew.-%, vorzugsweise höchstens etwa 40 Gew.-% und besonders bevorzugt höchstens etwa 30 Gew.-%, bezogen auf das Gewicht des Tablettenkerns.

Gemäss einer besonders bevorzugten Ausführungsform, enthält die erfindungsgemässe Tablettenformulierung als Hilfsstoffkomponente Natriumhydrogencarbonat und/oder Kaliumhydrogencarbonat und nicht vernetztes Polyvinylpyrrolidon. Vorzugsweise kann die Formulierung, bezogen auf das Gewicht des Tablettenkerns, etwa 5 bis 15 Gew.-%, insbesondere etwa 5 bis 10 Gew.-%, an nicht vernetztem Polyvinylpyrrolidon und etwa 7 bis 20 Gew.-%, insbesondere etwa 12 bis 18 Gew.-%, an Natriumhydrogencarbonat und/oder Kaliumhydrogencarbonat enthalten. Vorzugsweise enthält der Tablettenkern keine weiteren Hilfsstoffe, d.h. der Tablettenkern kann vorzugsweise aus dem Ibuprofen-Natrium-Hydrat, nicht vernetztem Polyvinylpyrrolidon und Natrium- und/oder Kaliumhydrogencarbonat bestehen.

Gewünschtenfalls kann die Tablettiermischung als Hilfsstoff auch ein Tensid wie Natriumdodecylsulfat enthalten. Der Anteil an Tensid, falls vorhanden, liegt jedoch in der Regel nicht über etwa 2 Gew.-% und kann typischerweise etwa 0,1 bis 2 Gew.-%, beispielsweise etwa 1 Gew.-%, bezogen auf das Gewicht des Tablettenkerns betragen. Der Zusatz eines Tensids ist jedoch in der Regel nicht erforderlich, weshalb die erfindungsgemässen Tablettenkern vorzugsweise tensidfrei sein können. Vorzugsweise kann daher die Hilfsstoffkomponente aus basischem Hilfsstoff und/oder neutralem bis schwach saurem, die Verpressbarkeit verbesserndem Füllstoff bestehen, d.h. der Tablettenkern besteht vorzugsweise aus dem Ibuprofen-Natrium-Hydrat und basischem Hilfsstoff und/oder neutralem bis schwach saurem, die Verpressbarkeit verbesserndem Füllstoff.

Liegt der Wassergehalt des Ibuprofen-Natrium-Hydrats unter 11 Gew.-%, d.h. im Bereich zwischen 8 und 11 Gew.-%, ist in der Regel ein vergleichsweise hoher Hilfsstoffanteil angezeigt, um der Verschlechterung der Eigenschaften des Ibuprofen-Natrium-Hydrats entgegenzuwirken. In diesem Falle ist daher im allgemeinen ein Anteil an Hilfsstoff, insbesondere Füllstoff und/oder basischer Hilfsstoff, von etwa 30 bis 50 Gew.-%, bezogen auf das Gewicht des Tablettenkerns, zu bevorzugen.

Die erfindungsgemässen Tabletten können den Wirkstoff Ibuprofen-Natrium-Hydrat in üblichen Dosierungen enthalten, wobei aufgrund des geringen Hilfsstoffanteils auch hohe Dosen möglich sind. Die erfindungsgemässen Tabletten können daher beispielsweise etwa 128 mg bis 1024 mg an Ibuprofen-Natrium-Hydrat (entsprechend 100 mg bis 800 mg Ibuprofen) enthalten, wobei Dosierungen im Bereich von etwa 256 mg bis 768 mg, insbesondere etwa 256 bis 512 mg im allgemeinen bevorzugt sind.

Die erfindungsgemässen Tablettenformlierungen können vorzugsweise mit einem Zucker- oder Filmcoating überzogen sein, wobei sich als Überzugsmaterialien grundsätzlich alle üblichen Zucker- und Filmüberzugsmaterialien eignen. Die Dicke des Überzugs ist nicht kritisch; im allgemeinen beträgt jedoch der Anteil des Überzugs, bezogen auf das Gewicht des Tablettenkern, lediglich etwa 1 bis 10 Gew.-%, vorzugsweise etwa 3 bis 6 Gew.-%.

Die erfindungemässen Tabletten können dadurch hergestellt werden, dass man das Ibuprofen-Natrium-Hydrat, allenfalls im Gemisch mit Hilfsstoff, zu Tablettenkernen verpresst und, gewünschtenfalls, die Tablettenkerne mit einem Zucker- oder Filmcoating überzieht. Die Tablettierung kann in an sich bekannter Weise auf üblichen Tablettenpressen erfolgen. Desgleichen kann ein allfälliger Zucker- oder Filmüberzug in an sich bekannter Weise nach üblichen Methoden aufgetragen werden. Bei der Herstellung sollte jedoch darauf geachtet werden, dass der Wassergehalt des Ibuprofen-Natrium-Hydrats in den oben angegebenen Bereichen liegt.

Im allgemeinen ist es bevorzugt, das Ibuprofen-Natrium-Hydrat vor der Tablettierung, allenfalls zusammen mit dem Hilfsstoff oder einem Teil des Hilfsstoffes, trocken zu granulieren. Weist das Ibuprofen-Natrium-Hydrat ein Schüttvolumen von mehr als 0,35 ml/g auf, kann gewünschtenfalls auf eine Granulierung verzichtet werden. Zur Bestimmung des Schüttvolumens wird ein 250 ml Messzylinder mit einer exakt gewogenen Menge Substanz, ohne zu schütteln, vorsichtig und langsam befüllt. Am Ende wird die eingeschüttete Substanz, falls nötig, mit einem Haarpinsel an der Oberfläche der Säule eingeebnet und das von der Substanz eingenommene Volumen abgelesen. Das Schüttvolumen ist der Quotient aus dem abgelesenen Volumen und der Masse der eingebrachten Substanz.

Bei Verwendung von Hilfsstoffen, insbesondere Füllstoff und/ oder basischer Hilfsstoff, können diese vor dem Granulieren oder erst der Endmischung direkt vor der Tablettierung zugemischt werden oder auch ein Teil der Hilfsstoffe bei der Granulierung eingesetzt und der Rest der Endmischung zugesetzt werden. Enthält die Tablette sowohl Füllstoff als auch basischen Hilfsstoff ist es aber in der Regel bevorzugt, den Füllstoff bereits bei der Granulation und den basischen Hilfsstoff erst der Endmischung zuzusetzen.

Die Erfindung betrifft ebenfalls eine Methode zur Erzielung einer beschleunigt einsetzenden analgetischen Wirkung, umfassend die Herstellung der erfindungsgemässen Tablette und deren Verabreichung an einen Schmerzpatienten.

Die Erfindung wird durch die nachfolgenden Beispiele weiter veranschaulicht. In den Beispielen bezeichnet Kollidon CL (Hoechst, Deutschland) ein quervernetztes, wasserunlösliches Polyvinylpyrrolidon, Povidone K25-K90 (BASF, Deutschland) wasserlösliche, nicht vernetzte Polyvinylpyrrolidone, Dimethicone (Wacker, Deutschland) ein Silikonöl, Hypromellose 2910, 6 und 15 mPas (Shin Etsu, Japan) eine wasserlösliche Hydroxypropylmethylcellulose, Magrogol 4000 und Magrogol 6000 (Hoechst, Deutschland) hochpolymeres, wachsartiges und wasserlösliches Polyethylenglycol mit einem mittleren Molekulargewicht von 4000 bzw. 6000, Titandioxid (Schweizerhalle, Schweiz) ein wasserunlösliches Weisspigment.

### Beispiel 1

a) 256,25 kg Ibuprofen-Natrium-Dihydrat wurden in einem konventionellen Mischer mit 25,0 kg Povidone K25 10 Minuten homogen gemischt. Diese Mischung wurde auf einem Walzenkompaktor (Roller compactor) verdichtet, und das verdichtete Material über ein Sieb mit der Maschenweite 1,0 mm gebrochen. Anteile mit einer Korngrösse unter 0,25 mm wurden erneut kompaktiert und gebrochen.
   49,75 kg Natriumhydrogencarbonat, gesiebt durch ein Sieb mit Maschenweite 0,71 mm, wurden in einem konventionellen Mischer mit dem kompaktierten Material 10 Minuten gemischt. Die erhaltene Endmischung wurde auf einer Rundläuferpresse (rotary press) mit 16 Stempeln bei einer durchschnittlichen Stundenleistung von 50'000 Tabletten abgepresst. Die erhaltenen ovalen, bikonvexen Tabletten hatten ein Gewicht von 331 mg, eine Länge von 11,7 mm, eine Breite von 7,7 mm und eine Höhe von 4,6 mm.
   Zur Bestimmung der Härte der Tabletten wurde die zum Brechen der Tablette zwischen den motorisierten Backen eines Schleuniger-Härtetesters benötigte Kraft gemessen. Die durchschnittliche Härte (Mittelwert aus 10 Messungen) betrug 78 N.
   Die Zerfallszeit der Tabletten wurde mittels der in der Europäischen Pharmacopöe, 4. Edition, Kapitel 2.9.1, Seite 191 beschriebenen Zerfallsmethode unter Verwendung von Wasser (pH etwa 7) als Zerfallsmedium gemessen. Die durchschnittliche Zerfallszeit der Tabletten (Mittelwert aus 6 Messungen) betrug 5,2 Minuten.
b) 331 kg der erhaltenen Tabletten wurden in einen Glatt-Coater geladen und bei einer Produkttemperatur von 35°C bis 42°C mit einer Lösung von 3,5 kg Hypromellose 2910, 0,75 kg Lactose-Monohydrat und 0,75 kg Magrogol 6000 in 10 kg Wasser und 40 kg Ethanol (96%) besprüht und dabei isoliert. Unter den gleichen Bedingungen wurden die isolierten Filmtablettenkerne mit einer Suspension von 2,8 kg Hypromellose 2910, 3,6 kg Lactose-Monohydrat, 1,0 kg Magrogol 4000 und 2,6 kg Titandioxid in 56 kg Wasser und 24 kg Ethanol (96%) besprüht. Die getrockneten Filmtabletten wurden mit einer Polierlösung aus 2 kg Magrogol 6000 und 17 kg Wasser behandelt. Das Endgewicht der Filmtabletten betrug 348 mg.

### Beispiel 2

Wie in Beispiel 1 beschrieben, wurden 331 kg der Tablettier-Endmischung hergestellt. Diese wurde in analoger Weise zu Beispiel 1 zu oblongen, bikonvexen Tabletten mit einseitiger Teilkerbe abgepresst, und die erhaltenen Tabletten wie in Beispiel 1 beschrieben zu Filmtabletten verarbeitet. Die Tablettenkerne hatten ein Gewicht von 662 mg, eine Länge von 17,3 mm, eine Breite von 8,3 mm, eine Höhe von 5,0 mm und einen Gehalt an Ibuprofen-Natrium-Dihydrat von 513 mg (ensprechend 400 mg Ibuprofen-Säure); die durchschnittliche Härte betrug 98 N und die durchschnittliche Zerfallszeit 5,7 Minuten. Das Endgewicht der Filmtabletten betrug 696 mg.

### Beispiele 3-50

a) In analoger Weise zu Beispiel 1a wurden die in Tabelle 1 aufgeführten Tablettenformulierungen hergestellt.
Zur Herstellung des Granulats wurden dem Ibuprofen-Natrium-Hydrat in einem konventionellem Mischer innert 10 Minuten allfällige zur Trockengranulation verwendete Exzipientien (Hilfsstoffe A) zugemischt, die erhaltene Mischung bzw. das ohne Hilfsstoffe verwendete Ibuprofen-Natrium-Hydrat auf einem Walzenkompaktor (Roller compactor) verdichtet, das verdichtete Material über eine Sieb mit der Maschenweite 1,0 mm gebrochen, und Anteile mit einer Korngrösse unter 0,25 mm erneut kompaktiert und gebrochen. In Beispiel 41 wurde ein Ibuprofen-Natrium-Hydrat mit einer mittleren Korngrösse von 0,1-0,2 mm und einem Schüttvolumen von über 0,35 g/ml verwendet und das erhaltene Ibuprofen-Natrium-Hydrat/Maltodextrin-Gemisch nicht kompaktiert, sondern direkt zur Tablettierung verwendet. In den Beispielen 28-30 wurde ein Granulat mit einer Korngrösse von 0,25-1,25 mm (Beispiel 28), 0-0,25 mm (Beispiel 29) bzw. 0-1,25 mm (Beispiel 30) hergestellt und zur Tablettierung verwendet. Der Wassergehalt des verwendeten Ibuprofen-Natrium-Hydrats wurde jeweils als Trockenverlust bei einer Trocknung bei 105°C bestimmt.
Das erhaltene Granulat (Korngrösse im Bereich von 0,25 bis 1,0 mm, sofern nicht anders angegeben) wurde in einem konventionellen Mischer 10 Minuten mit allfälligen Hilfsstoffen (Hilfsstoffe B) gemischt. Die erhaltene Endmischung (bzw. das Granulat selbst, sofern kein Hilfsstoff B eingesetzt wurde) wurde auf einer Rundläuferpresse (rotary press) mit 16 Stempeln bei einer durchschnittlichen Stundenleistung von 40'000-60'000 Tabletten abgepresst. Die erhaltenen ovalen, bikonvexen Tabletten hatten ein Gewicht von 300-350 mg, eine Länge von 11,7 mm, eine Breite von 7,7 mm und eine Höhe von etwa 4,6 mm für das verwendete Stempelwerkzeug.
Der Wassergehalt des verwendeten Ibuprofen-Natrium-Hydrats, der Anteil des Ibuprofen-Natium-Hydrats in der Tablettenformulierung sowie die verwendeten Hilfsstoffe A und B und deren Anteile in der Tablettenformulierung sind in Tabelle 1 zusammengestellt. 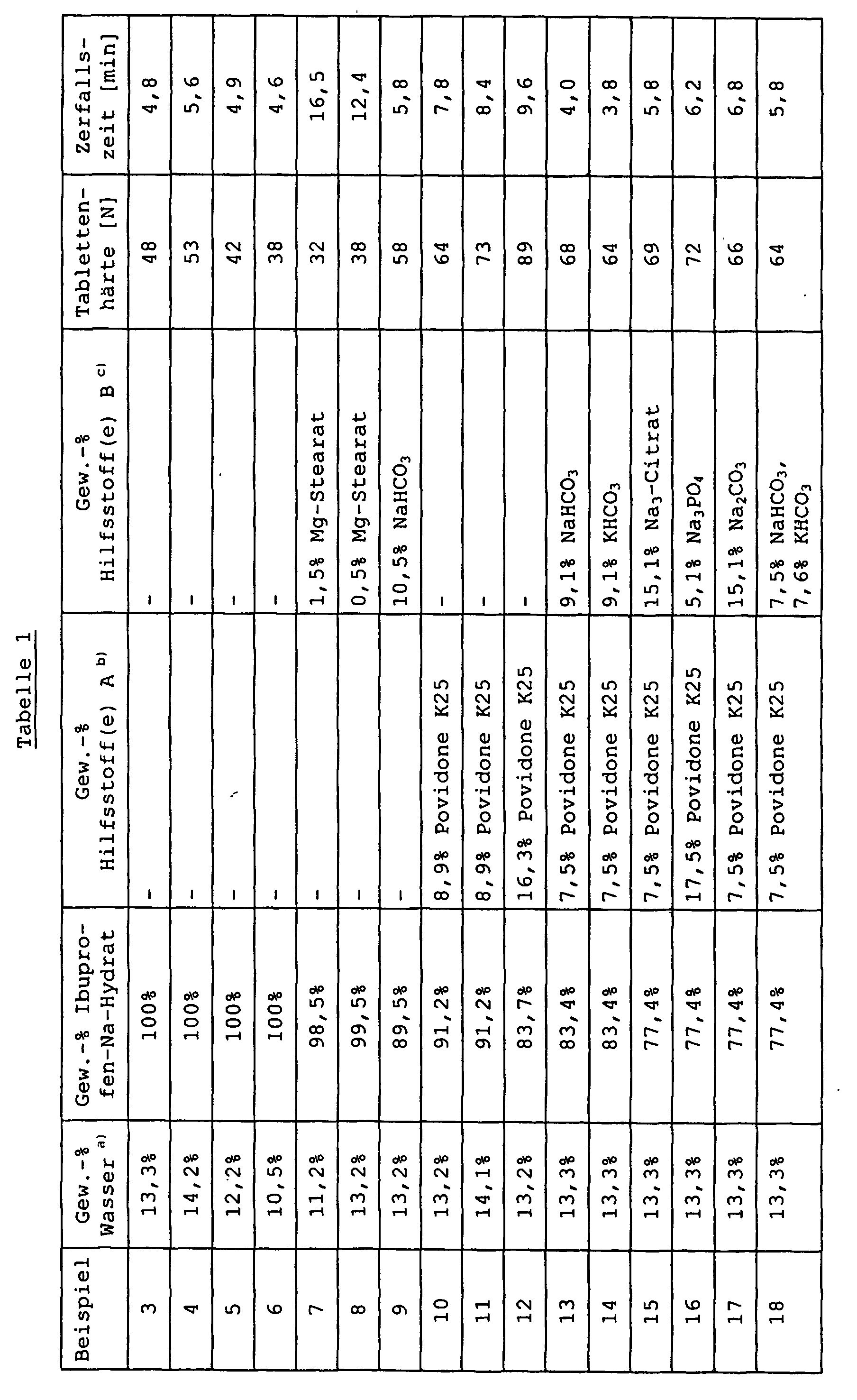 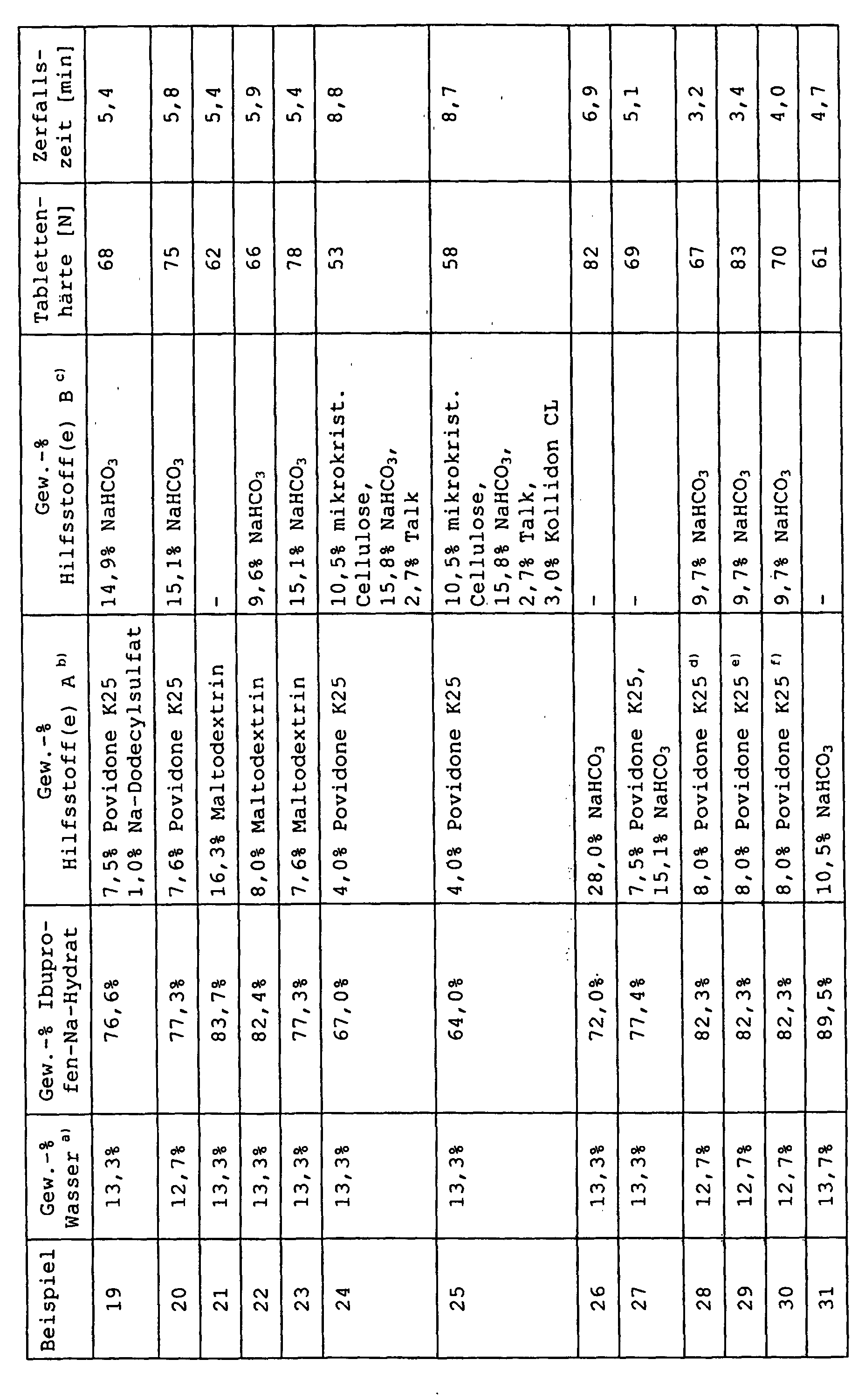 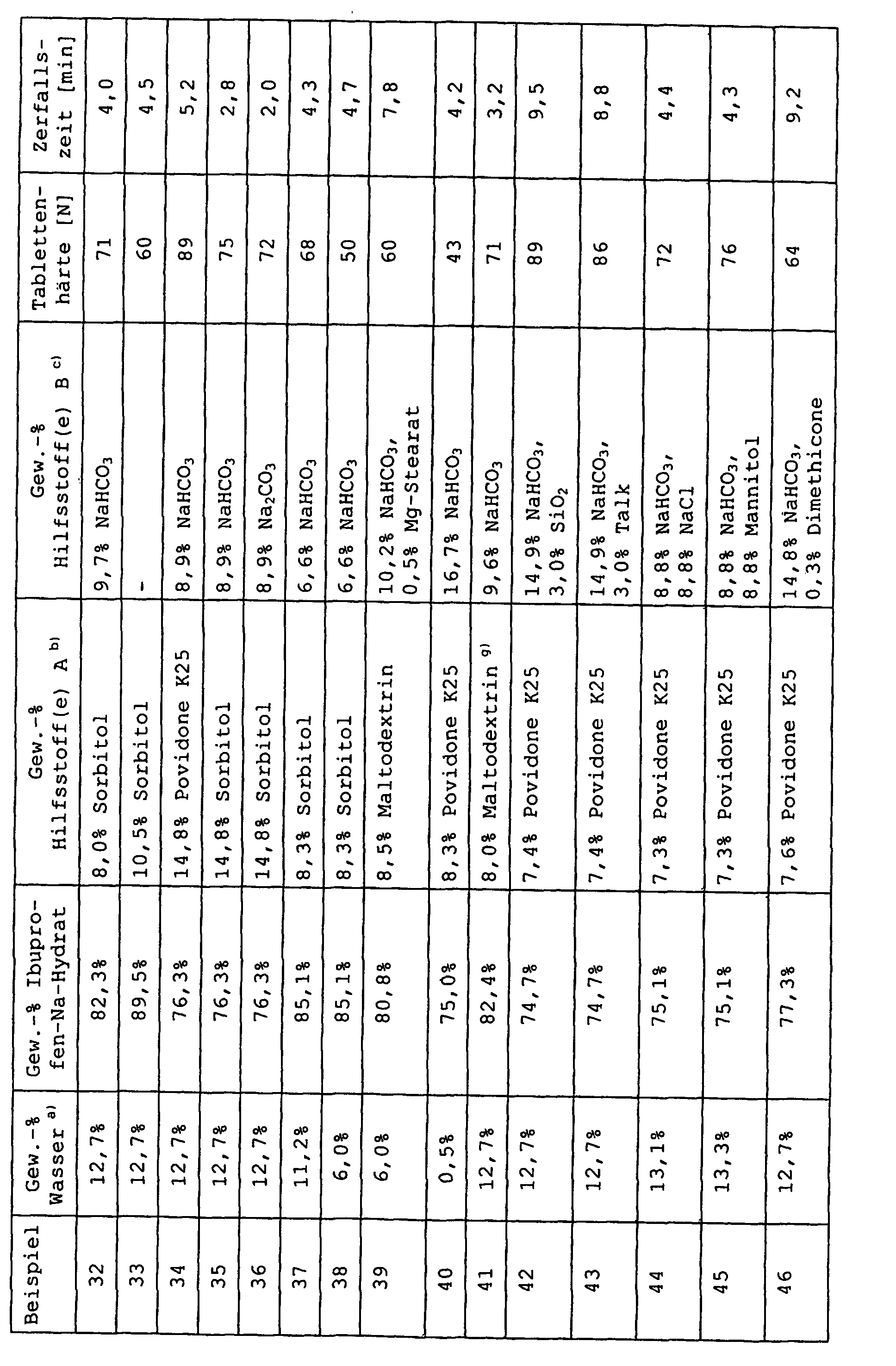 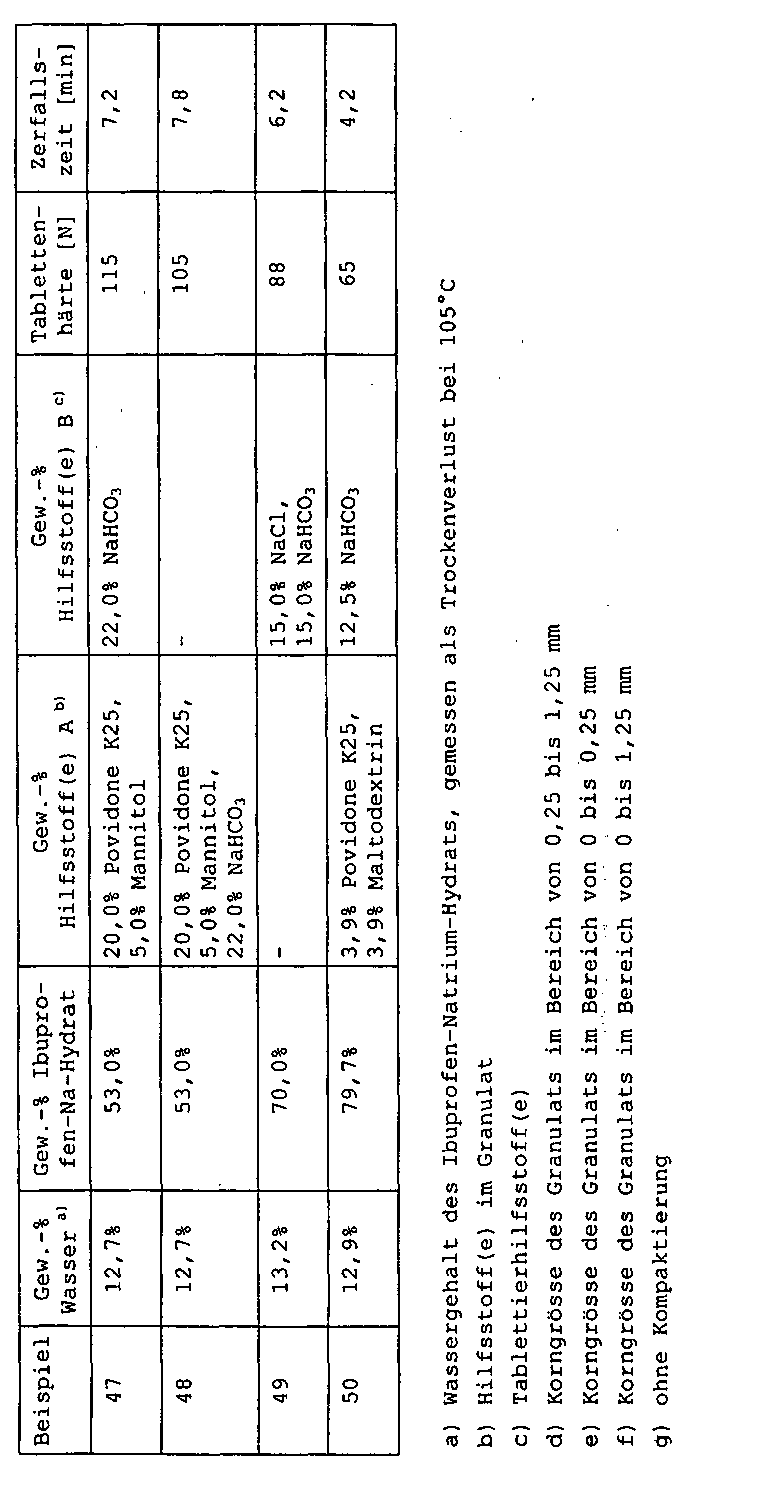
Zur Bestimmung der Härte der Tabletten (crushing strength) wurde die zum Brechen der Tablette zwischen den motorisierten Backen eines Schleuniger-Härtetesters benötigte Kraft gemessen. Die in Tabelle 1 angegebenen Werte sind jeweils Mittelwerte aus 10 Messungen.
Die Zerfallszeit der Tabletten wurde mittels der in der Europäischen Pharmacopöe, 4. Edition, Kapitel 2.9.1, Seite 191 beschriebenen Zerfallsmethode unter Verwendung von Wasser (pH etwa 7) als Zerfallsmedium gemessen. Die in Tabelle 1 angegebenen Zerfallszeiten sind jeweils Mittelwerte aus 6 Messungen.
Die Formulierung gemäss Beispiel 40 erwies sich als stark an den Tablettierwerkzeugen klebend mit starker Neigung zum Deckeln (capping). Deckelneigung wurde auch in den Beispielen 7 und 39 und ferner teilweise auch in den Beispielen 6, 37 und 38 beobachtet, wobei Beispiele 6, 38 und 39 zudem an den Tablettierwerkzeugen klebende Formulierungen ergaben und die Formulierung von Beispiel 7 teilweise klebend war; beide Effekte waren aber deutlich weniger ausgeprägt als im Beispiel 40. Die Formulierungen gemäss den Beispielen 42 und 43 waren klebend (ohne Deckelneigung), was teilweise auch auf jene der Beispiele 5, 24, 25 und 46 zutraf. Die Formulierungen gemäss den Beispielen 3 und 21 erwiesen sich als nur sehr wenig klebend und zeigten keine Neigung zum Deckeln. Die Formulierungen gemäss den Beispielen 9-20, 22, 23, 26-36, 41, 44, 45 und 47-50 zeigten gute bis sehr gute Tabletteneigenschaften (Härte, Zerfallszeit, Friabilität, Aussehen der Tablettenoberfläche), wobei insbesondere jene der Beispiele 12, 15-20, 22, 23, 29, 34, 47 und 48 praktisch perfekte Tabletten ergaben. Die Oberflächen der Tabletten waren einwandfrei glatt, nahezu porenfrei und eigneten sich bestens für das Filmcoating.
Der Einfluss des Wassergehaltes zeigt sich besonders in den Beispielen 3-6, in denen der reine Wirkstoff verpresst wurde. Gute bis akzeptable Tabletten werden erhalten, wenn der Wassergehalt mindestens 11 Gew.-% beträgt. Sinkt der Wassergehalt unter diesen Wert, kleben die Tabletten zunehmend an den Stempelwerkzeugen, und die Tabletten erreichen nur noch geringe Bruchfestigkeiten und neigen zum Deckeln. Das Kleben an den Stempelwerkzeugen kann durch den Zusatz des hochwirksamen Antiklebemittels Magnesiumstearat in keiner Weise vermieden werden; durch diesen Zusatz wird vielmehr die Härte der Tabletten drastisch reduziert und die Zerfallszeit steigt deutlich über 10 Minuten, wie Beispiele 7 und 8 illustrieren. Auch bei Zusatz von Füllstoffen und basischen Hilfsstoffen kann der negative Einfluss eines ungenügenden Wassergehalts nur sehr begrenzt kompensiert werden, wie Beispiele 38-40 veranschaulichen.
Wie Beispiele 24 und 25 zeigen, sind die Ergebnisse von Tablettenformulierungen, die zusätzlich mikrokristalline Cellulose, Talk und gegebenenfalls das Sprengmittel Kollidon CL enthalten, schlechter als vergleichbare Beispiele ohne diese Zusätze. Die Tablettenhärten werden durch diese Zusätze nicht verbessert, und die Zerfallszeiten liegen bei etwa 9 Minuten.
Wie die übrigen Beispiele belegen, werden bei ausreichendem Wassergehalt und unter Verwendung eines oder mehrerer Füllstoffe und/oder basischer Hilfsstoffe Tabletten mit ausreichender mechanischer Festigkeit, Zerfallszeiten unter 10 Minuten, meist zwischen etwa 2 und 7 Minuten, und Tablettenhärten in Abhängigkeit der eingesetzten Hilfsstoffmenge zwischen etwa 50 und 120 N erreicht.
b) In analoger Weise zu Beispiel 1b wurden die in den Beispielen 4, 11, 13, 19-23, 30, 45, 47, 49 und 50 erhaltenen Tabletten mit einem Filmüberzug versehen. Das Endgewicht der Filmtabletten betrug etwa 317-367 mg. Ferner wurden auch Filmüberzüge erfolgreich realisiert, die als Filmbildner Carrageenan, Polyvinylalkohol und Hydroxypropylmethylcellulose neben den üblichen Weichmachern wie Polyethylenglykole, Triethylcitrat und Triacetin enthielten.
Die Bioverfügbarkeit der gemäss den Beispielen 19 und 22 erhaltenen Filmtabletten (nachfolgend als Beispiele 19b und 22b bezeichnet) wurde an 15 Probanden untersucht, wobei als Referenzformulierung Nurofen-Tabletten (Boots), enthaltend 200 mg Ibuprofen verwendet wurde. Die Probanden erhielten jeweils 2 Filmtabletten bzw. Dragees. Die Ergebnisse der Bioverfügbarkeitsstudie sind in Tabelle 2 zusammengestellt:

**Tabelle 2**

| | Beispiel 19b | Beispiel 22b | Nurofen |
|---|---|---|---|
| Cₘₐₓ (µg/ml) | 46,4 ± 8,8 | 47, 6 ± 8, 7 | 36,8 ± 9,4 |
| AUC_{0-∞} (ng x h/ml) | 135, 6 ± 23,5 | 127,5 ± 25,5 | 130, 7 ± 26, 9 |
| tₘₐₓ (h) | 0, 67 ± 0, 4 | 0, 62 ± 0,3 | 1,4 ± 1,1 |

Da Ibuprofen bzw. die Ibuprofen-Salze im gesamten Intestinaltrakt resorbiert werden, ist es nicht verwunderlich, dass alle drei Präparate fast die gleiche Bioverfügbarkeit aufweisen. An den Cₘₐₓ-Werten ist hingegen ersichtlich, dass die erfindungsgemässen Formulierungen höhere maximale Blutspiegel ergeben. Besonders auffällig ist der grosse Unterschied in den zur Erreichung der maximalen Blutspiegel beobachteten Zeiten tₘₐₓ. Die erfindungsgemässen Formulierungen sind dem Referenzmuster deutlich überlegen. Es kommt zu einem wesentlich schnelleren Blutspiegelanstieg und das Maximum wird ca. 45 Minuten früher erreicht. Dies ist für ein Schmerzmittel von grosser Bedeutung. Ein zu spätes Erreichen des maximalen Blutspiegels kann den Patienten dazu verleiten, eine weitere Tablette einzunehmen, da die Schmerzbefreiung zu spät einsetzt.

### Beispiel 51 (Dissolution-Test)

Die Wirkstofffreisetzung aus den in den Beispielen 3-50 erhaltenen Tabletten und Filmtabletten wurde mittels der in der Europäischen Pharmacopöe, 4. Edition, Kapitel 2.9.3, Seite 194 beschriebenen Methode (Paddle-Gerät) in den folgenden drei Medien untersucht:
- 1000 ml einer 0,1 M Salzsäure (künstlicher Magensaft, pH 1,2);
- 1000 ml McIlvain-Puffer (pH 3,5), hergestellt aus 702 ml 0,1 M wässriger Zitronensäurelösung und 298 ml 0,2 M wässriger Na₂HPO₄-Lösung;
- 1000 ml USP-Puffer (pH 7,2), hergestellt aus 50 ml 0,2 M wässriger KH₂PO₄-Lösung und 34,7 ml 0,2 M wässriger NaOH-Lösung, aufgefüllt mit Wasser auf 1000 ml.

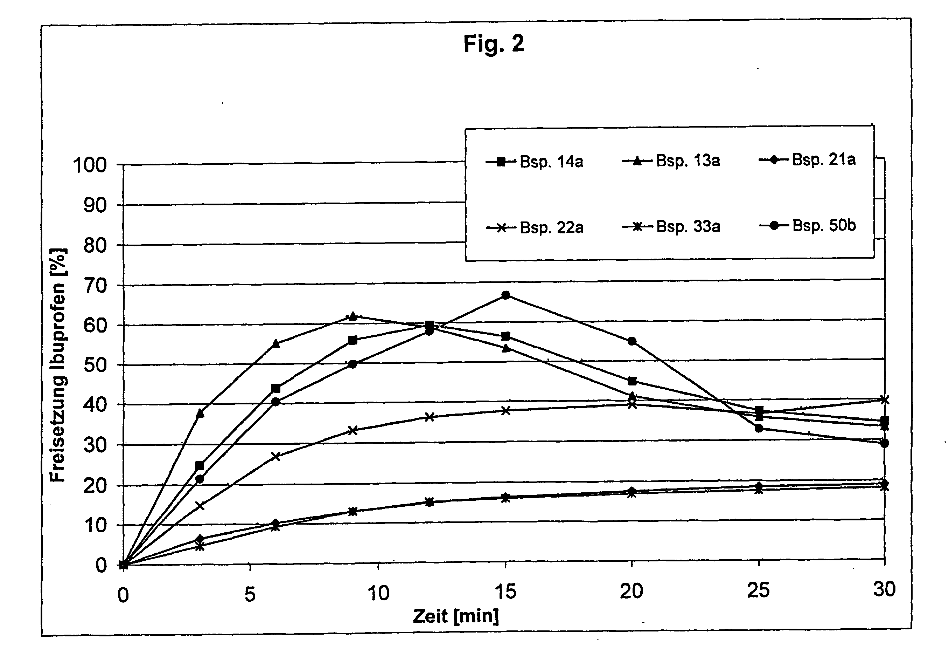

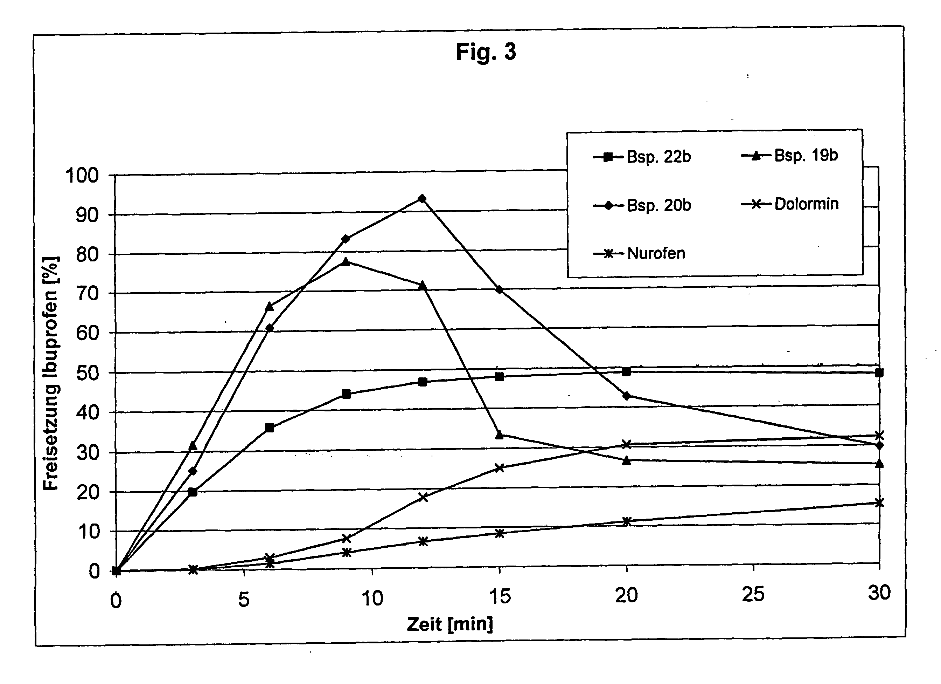

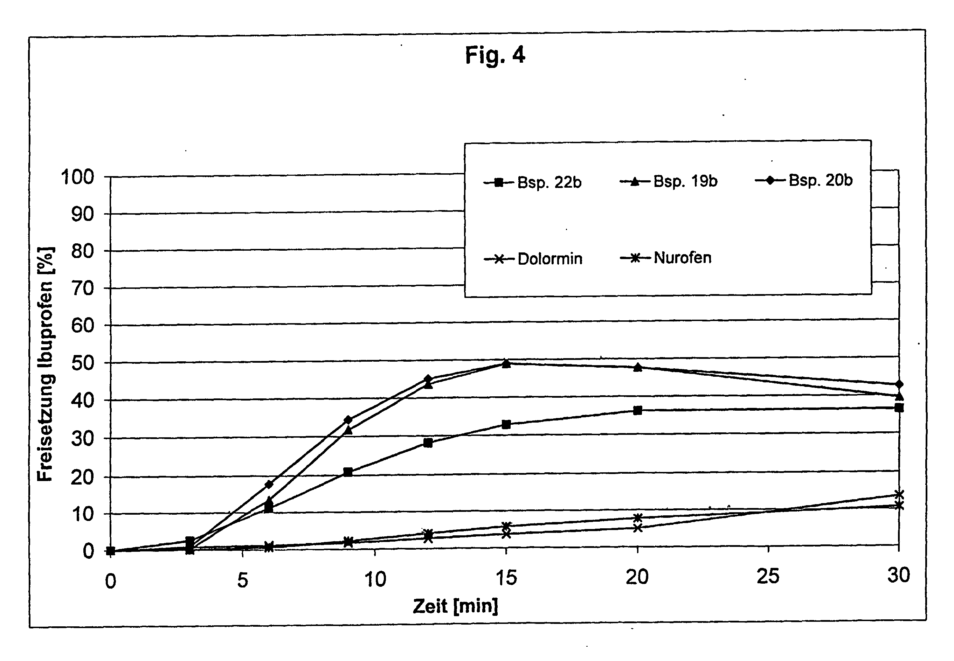

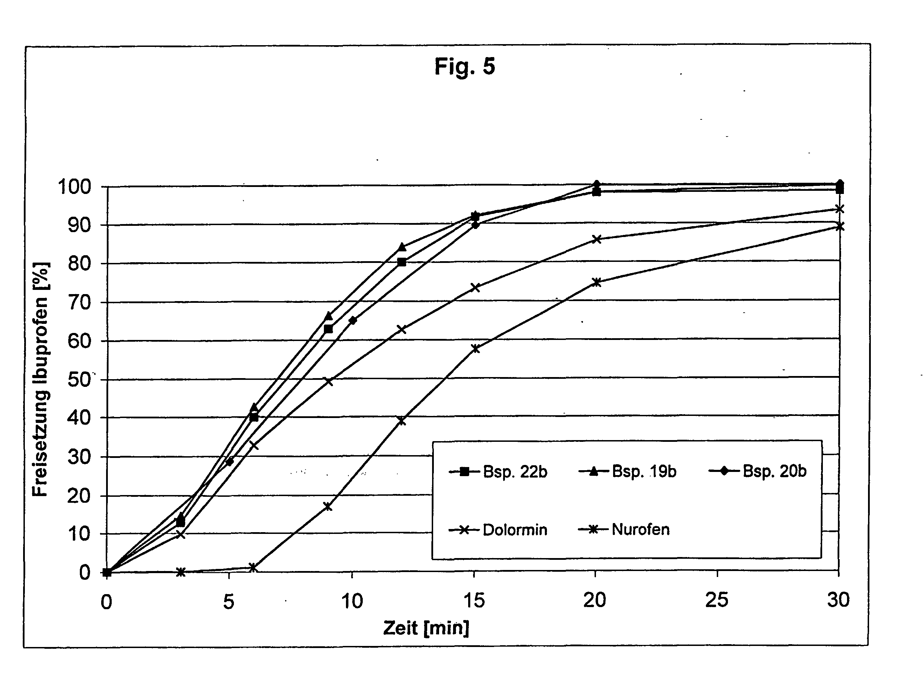

Die Auflösungsprofile einiger Formulierungen sind zur Veranschaulichung in Fig. 2-5 graphisch dargestellt. Fig. 2 zeigt die nach der Paddle-Methode in 0,1 M Salzsäure bei 50 UpM gemessenen Auflösungsprofile der nicht überzogenen Tabletten (Tablettenkerne) gemäss den Beispielen 13, 14, 21, 22 und 33 (nachfolgend und in Fig. 2 als Beispiele 13a, 14a, 21a, 22a bzw. 33a bezeichnet) und der Filmtablette gemäss Beispiel 50 (nachfolgend und in Fig. 2 als Beispiel 50b bezeichnet). Fig. 3-5 zeigen die nach der Paddle-Methode in den obigen Medien gemessenen Auflösungsprofile der Filmtabletten gemäss den Beispielen 19, 20 und 22 (nachfolgend und in Fig. 3-5 als Beispiele 19b, 20b bzw. 22b bezeichnet) und zum Vergleich die entsprechenden Auflösungsprofile der im Handel erhältlichen Präparate Dolormin (Woelm Pharma, Deutschland), einer Filmtablette, enthaltend 342 mg Ibuprofen-Lysinat, und Nurofen (Boots, Grossbritannien), eines mit Zucker überzogenen Dragees, enthaltend 200 mg Ibuprofen in Form der Säure; Fig. 3 zeigt die Auflösungsprofile in 0,1 M Salzsäure bei 100 UpM, Fig. 4 die Auflösungsprofile in McIlvain-Puffer bei 100 UpM und Fig. 5 die Auflösungsprofile in USP-Puffer bei 50 UpM.

Ibuprofen ist eine organische Säure mit einer stark pH-abhängigen Löslichkeit. In dem pH-Bereich von 1-5 liegt die Löslichkeit deutlich unter 0,1 g/l. Erst ab pH 6 steigt sie infolge Salzbildung sehr stark an und erreicht pH 7 einen Wert von etwa 20 g/l. Wird die in-vitro-Freisetzung bei pH 7,2 gemessen, ist es daher nicht verwunderlich, dass für die Nurofen-Tablette, die Ibuprofen in Form der Säure enthält, ebenfalls eine rasche Wirkstofffreisetzung beobachtet wird; selbst bei pH 7,2 ist jedoch die Wirkstofffreisetzung aus den erfindungsgemässen Filmtabletten rascher als aus der Ibuprofenlysinat-Filmtablette Dolormin und insbesondere der Ibuprofen-Filmtablette Nurofen. Dieser Unterschied bei pH 7,2 vermag jedoch nicht zu erklären, warum maximale Blutspiegel mit den erfindungsgemässen Formulierungen etwa 45 Minuten schneller erreicht werden als mit Nurofen.

Eine Erklärung für den erfindungsgemäss erreichbaren, wesentlich rascheren Blutspiegelanstieg bietet jedoch das Auflösungsverhalten bei sauren pH-Werten (Fig. 2-4). Aufgrund der geringen Löslichkeit des Ibuprofens bei pH-Werten unter 5 und des begrenzten Volumens des Auslösungsmediums wurde der Wirkstoff in diesen Versuchen jeweils nicht vollständig aufgelöst. Für Dolormin und Nurofen wurde eine vergleichsweise langsame, allmähliche Auflösung beobachtet, wie in Fig. 3 und 4 veranschaulicht. Die erfindungsgemässen Formulierungen zeigten demgegenüber ein wesentlich verbessertes Auflösungsverhalten, wobei die Filmtabletten gemäss den Beispielen 20b und 22b insbesondere bei pH 1,2 zur Bildung stark übersättiger Lösungen neigten (ohne dass dabei die pH-Werte der Dissolutionsmedien verändert wurden). Der nach etwa 10-20 Minuten einsetzende Abfall der Kurven ist eine Folge der allmählichen Kristallisation des Ibuprofens, womit die Übersättigung allmählich abgebaut wird. Es wird vermutet, dass Übersättigungsphänomene auch bei der beobachteten, ausgezeichneten in-vivo-Resorption eine wichtige Rolle spielen und dass Übersättigungen durch die komplexe Zusammensetzung des Magen- und Darmsaftes sogar deutlich besser stabilisiert werden dürften als unter in-vitro-Bedingungen.

## Patentansprüche

1. Nicht-brausende Tablette zur oralen Verabreichung von Ibuprofen-Natrium, umfassend einen Tablettenkern und gewünschtenfalls einen Zucker- oder Filmüberzug auf dem Tablettenkern, worin der Tablettenkern, bezogen auf das Gewicht des Tablettenkerns, aus 50 bis 100 Gew.-% Ibuprofen-Natrium-Hydrat und 50 bis 0 Gew.-% Hilfsstoffkomponente besteht und kein Schmiermittel und kein Sprengmittel enthält, wobei das Ibuprofen-Natrium-Hydrat einen Wassergehalt von 8 bis 16 Gew.-%, bezogen auf das Hydrat und gemessen als Trocknungsverlust bei 105°C, aufweist.

2. Tablette nach Anspruch 1, worin der Wassergehalt des Ibuprofen-Natrium-Hydrates 11 bis 16 Gew.-%, bezogen auf das Hydrat, beträgt.

3. Tablette nach Anspruch 1 oder 2, worin der Wassergehalt des Ibuprofen-Natrium-Hydrates 12,5 bis 15 Gew.-%, bezogen auf das Hydrat, beträgt.

4. Tablette nach einem der Ansprüche 1 bis 3, worin das Ibuprofen-Natrium-Hydrat in einer Menge von 50 bis 99,9 Gew.-%, bezogen auf das Gewicht des Tablettenkerns vorliegt.

5. Tablette nach einem der Ansprüche 1 bis 4, worin das Ibuprofen-Natrium-Hydrat in einer Menge von mindestens 60 Gew.-%, bezogen auf das Gewicht des Tablettenkerns, vorliegt.

6. Tablette nach einem der Ansprüche 1 bis 5, worin das Ibuprofen-Natrium-Hydrat in einer Menge von 60 bis 93 Gew.-%, bezogen auf das Gewicht des Tablettenkerns, vorliegt.

7. Tablette nach einem der Ansprüche 1 bis 6, worin das Ibuprofen-Natrium-Hydrat in einer Menge von mindestens 70 Gew.-%, bezogen auf das Gewicht des Tablettenkerns, vorliegt.

8. Tablette nach einem der Ansprüche 1 bis 7, worin das Ibuprofen-Natrium-Hydrat in einer Menge von 70 bis 85 Gew.-%, bezogen auf das Gewicht des Tablettenkerns, vorliegt.

9. Tablette nach einem der Ansprüche 1 bis 8, worin die Hilfsstoffkomponente einen oder mehrere basische Hilfsstoffe umfasst.

10. Tablette nach einem der Ansprüche 1 bis 9, worin die Hilfsstoffkomponente einen oder mehrere wasserlösliche, basische Hilfsstoffe umfasst.

11. Tablette nach einem der Ansprüche 1 bis 10, worin die Hilfsstoffkomponente einen oder mehrere basische Hilfsstoffe, ausgewählt aus basischen Alkalimetall-, Erdalkalimetall- und Ammoniumsalzen und basischen Aminosäuren, umfasst.

12. Tablette nach einem der Ansprüche 1 bis 11, worin die Hilfsstoffkomponente einen oder mehrere basische Hilfsstoffe, ausgewählt aus Natriumhydrogencarbonat, Kaliumhydrogencarbont, Natriumcarbonat, Kaliumcarbonat, Trinatriumcitrat und Trinatriumphosphat, umfasst.

13. Tablette nach einem der Ansprüche 1 bis 12, worin die Hilfsstoffkomponente mindestens einen basischen Hilfsstoff, ausgewählt aus Natriumhydrogencarbonat und Kaliumhydrogencarbonat, umfasst.

14. Tablette nach einem der Ansprüche 9 bis 13, worin der Anteil an basischem Hilfsstoff, bezogen auf das Gewicht des Tablettenkerns, 5 bis 30 Gew.-% beträgt.

15. Tablette nach einem der Ansprüche 9 bis 14, worin der Anteil an basischem Hilfsstoff, bezogen auf das Gewicht des Tablettenkerns, 6 bis 25 Gew.-% beträgt.

16. Tablette nach einem der Ansprüche 1 bis 15, worin die Hilfsstoffkomponente einen oder mehrere neutrale bis schwach saure, die Verpressbarkeit verbessernde Füllstoffe umfasst.

17. Tablette nach einem der Ansprüche 1 bis 16, worin die Hilfsstoffkomponente einen oder mehrere wasserlösliche, neutrale bis schwach saure, die Verpressbarkeit verbessernde Füllstoffe umfasst.

18. Tablette nach einem der Ansprüche 1 bis 17, worin die Hilfsstoffkomponente einen oder mehrere Füllstoffe, ausgewählt aus Zuckern, Hexosen, hydrolysierten oder enzymatisch gespaltenen Stärken, Cyclodextrinen, nicht vernetztem Polyvinylpyrrolidon und neutralen bis schwach sauren Alkalimetall-, Erdalkalimetall- und Ammoniumsalzen, umfasst.

19. Tablette nach einem der Ansprüche 1 bis 18, worin die Hilfsstoffkomponente einen oder mehrere Füllstoffe, ausgewählt aus Hexosen, nicht vernetztem Polyvinylpyrrolidon, Maltodextrin und Natriumchlorid, umfasst.

20. Tablette nach einem der Ansprüche 1 bis 19, worin die Hilfsstoffkomponente nicht vernetztes Polyvinylpyrrolidon als Füllstoff umfasst.

21. Tablette nach einem der Ansprüche 16 bis 20, worin der Anteil an Füllstoff, bezogen auf das Gewicht des Tablettenkerns, 1 bis 25 Gew.-% beträgt.

22. Tablette nach einem der Ansprüche 16 bis 21, worin der Anteil an Füllstoff, bezogen auf das Gewicht des Tablettenkerns, 3 bis 20 Gew.-% beträgt.

23. Tablette nach einem der Ansprüche 1 bis 22, worin die Hilfsstoffkomponente einen oder mehrere basische Hilfsstoffe und einen oder mehrere neutrale bis schwach saure, die Verpressbarkeit verbessernden Füllstoffe umfasst.

24. Tablette nach einem der Ansprüche 1 bis 23, worin die Hilfsstoffkomponente mindestens einen basischen Hilfsstoff, ausgewählt aus Natriumhydrogencarbonat und Kaliumhydrogencarbonat, und nicht vernetztes Polyvinylpyrrolidon als Füllstoff umfasst.

25. Tablette nach einem der Ansprüche 1 bis 24, worin die Hilfsstoffkomponente, bezogen auf das Gewicht des Tablettenkerns, 5 bis 15 Gew.-% an basischem Hilfsstoff, ausgewählt aus Natriumhydrogencarbonat und Kaliumhydrogencarbonat, und 7 bis 20 Gew.-% an nicht vernetztes Polyvinylpyrrolidon als Füllstoff umfasst.

26. Tablette nach einem der Ansprüche 1 bis 25, worin die Hilfsstoffkomponente aus basischem Hilfsstoff und/oder neutralem bis schwach saurem, die Verpressbarkeit verbesserndem Füllstoff besteht.

27. Tablette nach Anspruch 1 oder 2, worin der Tablettenkern aus Ibuprofen-Natrium-Hydrat besteht, das Ibuprofen-Natrium-Hydrat einen Wassergehalt von 11 bis 16 Gew.-% aufweist, und die Härte der Tablette mindestens 30 N beträgt.

28. Tablette nach Anspruch 27, worin das Ibuprofen-Natrium-Hydrat einen Wassergehalt von 12,5 bis 15 Gew.-% aufweist.

29. Tablette nach Anspruch 27 oder 28, worin die Härte der Tablette mindestens 40 N beträgt.

30. Tablette nach einem der Ansprüche 1 bis 29, worin das Ibuprofen-Natrium-Hydrat in racemischer Form vorliegt.

31. Tablette nach einem der Ansprüche 1 bis 29, worin das Ibuprofen-Natrium-Hydrat in Form des S(+)-Ibuprofen-Natrium-Hydrates vorliegt.

32. Tablette nach einem der Ansprüche 1 bis 31, worin der Tablettenkern mit einem Zucker- oder Filmüberzug überzogen ist.

33. Tablette nach einem der Ansprüche 1 bis 32, worin der Tablettenkern mit einem Zucker- oder Filmüberzug in einer Menge von 1 bis 10 Gew.-%, bezogen auf das Gewicht des Tablettenkerns, überzogen ist.

34. Verfahren zur Herstellung einer nicht-brausenden Tablette zur oralen Verabreichung von Ibuprofen-Natrium, umfassend einen Tablettenkern und gewünschtenfalls einen Zucker- oder Filmüberzug auf dem Tablettenkern, worin der Tablettenkern, bezogen auf das Gewicht des Tablettenkerns, aus 50 bis 100 Gew.-% Ibuprofen-Natrium-Hydrat und 50 bis 0 Gew.-% Hilfsstoffkomponente besteht und kein Schmiermittel und kein Sprengmittel enthält, und wobei das Ibuprofen-Natrium-Hydrat einen Wassergehalt von 8 bis 16 Gew.-%, bezogen auf das Hydrat und gemessen als Trockungsverlust bei 105°C, aufweist, **dadurch gekennzeichnet, dass** man das Ibuprofen-Natrium-Hydrat, allenfalls im Gemisch mit Hilfsstoff, zu Tablettenkernen verpresst und, gewünschtenfalls, die Tablettenkerne mit einem Zucker- oder Filmüberzug überzieht.

## Claims

1. Non-effervescent tablet for oral administration of sodium ibuprofen comprising a tablet core and, if desired, a sugar or film coat on the tablet core, wherein the tablet core consists of 50 to 100% by weight of sodium ibuprofen hydrate and 50 to 0% by weight of auxiliary material component, based on the weight of the tablet core, and contains no lubricant and no disintegrant, the sodium ibuprofen hydrate having a water content of from 8 to 16% by weight of the hydrate, measured as the drying loss at 105°C.

2. Tablet as claimed in claim 1, wherein the water content of the sodium ibuprofen hydrate is 11 to 16 % by weight of the hydrate.

3. Tablet as claimed in claim 1 or 2, wherein the water content of the sodium ibuprofen hydrate is 12.5 to 15 % by weight of the hydrate.

4. Tablet as claimed in any one of claims 1 to 3, wherein the sodium ibuprofen hydrate is present in an amount of from 50 to 99.9% by weight, based on the weight of the tablet core.

5. Tablet as claimed in any one of claims 1 to 4, wherein the sodium ibuprofen hydrate is present in an amount of at least 60% by weight, based on the weight of the tablet core.

6. Tablet as claimed in any one of claims 1 to 5, wherein the sodium ibuprofen hydrate is present in an amount of from 60 to 93% by weight, based on the weight of the tablet core.

7. Tablet as claimed in any one of claims 1 to 6, wherein the sodium ibuprofen hydrate is present in an amount of at least 70% by weight, based on the weight of the tablet core.

8. Tablet as claimed in any one of claims 1 to 7, wherein the sodium ibuprofen hydrate is present in an amount of from 70 to 85% by weight, based on the weight of the tablet core.

9. Tablet as claimed in any one of claims 1 to 8, wherein the auxiliary material component comprises one or more basic auxiliary materials.

10. Tablet as claimed in any one of claims 1 to 9, wherein the auxiliary material component comprises one or more water soluble, basic auxiliary materials.

11. Tablet as claimed in any one of claims 1 to 10, wherein the auxiliary material component comprises one or more basic auxiliary materials, selected from basic alkali metal salts, basic alkaline earth metal salts, basic ammonium salts and basic amino acids.

12. Tablet as claimed in any one of claims 1 to 11, wherein the auxiliary material component comprises one or more basic auxiliary materials, selected from sodium hydrogen carbonate, potassium hydrogen carbonate, sodium carbonate, potassium carbonate, trisodium citrate and trisodium phosphate.

13. Tablet as claimed in any one of claims 1 to 12, wherein the auxiliary material component comprises at least one basic auxiliary material, selected from sodium hydrogen carbonate and potassium hydrogen carbonate.

14. Tablet as claimed in any one of claims 9 to 13, wherein the proportion of the basic auxiliary material is 5 to 30% by weight, based on the weight of the tablet core.

15. Tablet as claimed in any one of claims 9 to 14, wherein the proportion of the basic auxiliary material is 6 to 25% by weight, based on the weight of the tablet core.

16. Tablet as claimed in any one of claims 1 to 15, wherein the auxiliary material component comprises one or more neutral to weakly acidic fillers that improve the compressibility.

17. Tablet as claimed in any one of claims 1 to 16, wherein the auxiliary material component comprises one or more water soluble, neutral to weakly acidic fillers that improve the compressibility.

18. Tablet as claimed in any one of claims 1 to 17, wherein the auxiliary material component comprises one or more fillers, selected from sugars, hexoses, hydrolysed or enzymatically split starches, cyclodextrins, non-crosslinked polyvinylpyrrolidone, neutral to weakly acidic alkali metal salts, neutral to weakly acidic alkaline earth metal salts, and neutral to weakly acidic ammonium salts.

19. Tablet as claimed in any one of claims 1 to 18, wherein the auxiliary material component comprises one or more fillers, selected from hexoses, non-crosslinked polyvinylpyrrolidone, maltodextrin and sodium chloride.

20. Tablet as claimed in any one of claims 1 to 19, wherein the auxiliary material component comprises non-crosslinked polyvinylpyrrolidone as filler.

21. Tablet as claimed in any one of claims 16 to 20, wherein the proportion of the filler is 1 to 25% by weight, based on the weight of the tablet core.

22. Tablet as claimed in any one of claims 16 to 21, wherein the proportion of the filler is 3 to 20% by weight, based on the weight of the tablet core.

23. Tablet as claimed in any one of claims 1 to 22, wherein the auxiliary material component comprises one or more basic auxiliary materials and one or more neutral to weakly acidic fillers that improve the compressibility.

24. Tablet as claimed in any one of claims 1 to 23, wherein the auxiliary material component comprises at least one basic auxiliary material, selected from sodium hydrogen carbonate and potassium hydrogen carbonate, and non-crosslinked polyvinylpyrrolidone as filler.

25. Tablet as claimed in any one of claims 1 to 24, wherein the auxiliary material component comprises, based on the weight of the tablet core, 5 to 15% of basic auxiliary material, selected from sodium hydrogen carbonate and potassium hydrogen carbonate, and 7 to 20% of non-crosslinked polyvinylpyrrolidone as filler.

26. Tablet as claimed in any one of claims 1 to 25, wherein the auxiliary material component consists of basic auxiliary material and/or neutral to weakly acidic filler that improves the compressibility.

27. Tablet as claimed in claim 1 or 2, wherein the tablet core consists of sodium ibuprofen hydrate, the sodium ibuprofen hydrate has a water content of 11 to 16% by weight, and the hardness of the tablet is at least 30 N.

28. Tablet as claimed in claim 27, wherein the sodium ibuprofen hydrate has a water content of 12.5 to 15% by weight.

29. Tablet as claimed in claim 27 or 28, wherein the hardness of the tablet is at least 40 N.

30. Tablet as claimed in any one of claims 1 to 29, wherein the sodium ibuprofen hydrate is present in racemic form.

31. Tablet as claimed in any one of claims 1 to 29, wherein the sodium ibuprofen hydrate is present in the form of sodium S(+)-ibuprofen hydrate.

32. Tablet as claimed in any one of claims 1 to 31, wherein the tablet core is coated with a sugar or film coat.

33. Tablet as claimed in any one of the claims 1 to 32, wherein the tablet core is coated with a sugar or film coat in an amount of from 1 to 10% by weight, based on the weight of the tablet core.

34. Process for producing a non-effervescent tablet for oral administration of sodium ibuprofen comprising a tablet core and, if desired, a sugar or film coat on the tablet core, wherein the tablet core consists of 50 to 100% by weight sodium ibuprofen hydrate and 50 to 0% by weight auxiliary material component, based on the weight of the tablet core, and contains no lubricant and no disintegrant, and wherein the sodium ibuprofen hydrate has a water content of 8 to 16% by weight of the hydrate, measured as the drying loss at 105°C, **characterized in that** the sodium ibuprofen hydrate, or, where applicable, a mixture thereof with auxiliary material, is compressed into the tablet cores and, if desired, the tablet cores are coated with a sugar or film coat.

## Revendications

1. Comprimé non effervescent pour administration orale d'ibuprofène sodique, comprenant un comprimé nu et, si on le souhaite, une dragéification ou un pelliculage sur le comprimé nu, dans lequel le comprimé nu se compose de 50 à 100% en poids d'hydrate d'ibuprofène sodique et de 50 à 0% en poids d'adjuvant, sur la base du poids du comprimé nu, et ne contient ni lubrifiant ni agent de délitement, l'hydrate d'ibuprofène sodique ayant une teneur en eau allant de 8 à 16%, en poids, de l'hydrate, ladite teneur étant mesurée comme perte au séchage à 105°C.

2. Comprimé selon la revendication 1, dans lequel la teneur en eau de l'hydrate d'ibuprofène sodique est de 11 à 16% en poids de l'hydrate.

3. Comprimé selon la revendication 1 ou 2, dans lequel la teneur en eau de l'hydrate d'ibuprofène sodique est de 12,5 à 15% en poids de l'hydrate.

4. Comprimé selon l'une quelconque des revendications 1 à 3, dans lequel l'hydrate d'ibuprofène sodique est présent en une quantité allant de 50 à 99,9% en poids, sur la base du poids du comprimé nu.

5. Comprimé selon l'une quelconque des revendications 1 à 4, dans lequel l'hydrate d'ibuprofène sodique est présent en une quantité d'au moins 60% en poids, sur la base du poids du comprimé nu.

6. Comprimé selon l'une quelconque des revendications 1 à 5, dans lequel l'hydrate d'ibuprofène sodique est présent en une quantité allant de 60 à 93% en poids, sur la base du poids du comprimé nu.

7. Comprimé selon l'une quelconque des revendications 1 à 6, dans lequel l'hydrate d'ibuprofène sodique est présent en une quantité d'au moins 70% en poids, sur la base du poids du comprimé nu.

8. Comprimé selon l'une quelconque des revendications 1 à 7, dans lequel l'hydrate d'ibuprofène sodique est présent en une quantité allant de 70 à 85% en poids, sur la base du poids du comprimé nu.

9. Comprimé selon l'une quelconque des revendications 1 à 8, dans lequel l'adjuvant comprend un ou plusieurs adjuvants basiques.

10. Comprimé selon l'une quelconque des revendications 1 à 9, dans lequel l'adjuvant comprend un ou plusieurs adjuvants basiques, solubles dans l'eau.

11. Comprimé selon l'une quelconque des revendications 1 à 10, dans lequel l'adjuvant comprend un ou plusieurs adjuvants basiques, sélectionnés parmi les sels basiques de métaux alcalins, les sels basiques de métaux alcalino-terreux, les sels basiques d'ammonium et les acides aminés basiques.

12. Comprimé selon l'une quelconque des revendications 1 à 11, dans lequel l'adjuvant comprend un ou plusieurs adjuvants basiques, sélectionnés parmi l'hydrogénocarbonate de sodium, l'hydrogénocarbonate de potassium, le carbonate de sodium, le carbonate de potassium, le citrate de trisodium et le phosphate de trisodium.

13. Comprimé selon l'une quelconque des revendications 1 à 12, dans lequel l'adjuvant comprend au moins un adjuvant basique, sélectionné parmi l'hydrogénocarbonate de sodium et l'hydrogénocarbonate de potassium.

14. Comprimé selon l'une quelconque des revendications 9 à 13, dans lequel la proportion de l'adjuvant basique est de 5 à 30% en poids, sur la base du poids du comprimé nu.

15. Comprimé selon l'une quelconque des revendications 9 à 14, dans lequel la proportion de l'adjuvant basique est de 6 à 25% en poids, sur la base du poids du comprimé nu.

16. Comprimé selon l'une quelconque des revendications 1 à 15, dans lequel l'adjuvant comprend un ou plusieurs agents de remplissage neutres à faiblement acides qui améliorent la compressibilité.

17. Comprimé selon l'une quelconque des revendications 1 à 16, dans lequel l'adjuvant comprend un ou plusieurs agents de remplissage neutres à faiblement acides, solubles dans l'eau, qui améliorent la compressibilité.

18. Comprimé selon l'une quelconque des revendications 1 à 17, dans lequel l'adjuvant comprend un ou plusieurs agents de remplissage, sélectionnés parmi des sucres, des hexoses, des amidons hydrolysés ou dégradés par des enzymes, des cyclodextrines, la polyvinylpyrrolidone non réticulée, des sels de métaux alcalins neutres à faiblement acides, des sels de métaux alcalino-terreux neutres à faiblement acides et des sels d'ammonium neutres à faiblement acides.

19. Comprimé selon l'une quelconque des revendications 1 à 18, dans lequel l'adjuvant comprend un ou plusieurs agents de remplissage, sélectionnés parmi des hexoses, la polyvinylpyrrolidone non réticulée, la maltodextrine et le chlorure de sodium.

20. Comprimé selon l'une quelconque des revendications 1 à 19, dans lequel l'adjuvant comprend la polyvinylpyrrolidone non réticulée comme agent de remplissage.

21. Comprimé selon l'une quelconque des revendications 16 à 20, dans lequel la proportion d'agent de remplissage est de 1 à 25% en poids, sur la base du poids du comprimé nu.

22. Comprimé selon l'une quelconque des revendications 16 à 21, dans lequel la proportion d'agent de remplissage est de 3 à 20% en poids, sur la base du poids du comprimé nu.

23. Comprimé selon l'une quelconque des revendications 1 à 22, dans lequel l'adjuvant comprend un ou plusieurs adjuvants basiques et un ou plusieurs agents de remplissage neutres à faiblement acides qui améliorent la compressibilité.

24. Comprimé selon l'une quelconque des revendications 1 à 23, dans lequel l'adjuvant comprend au moins un adjuvant basique, sélectionné parmi l'hydrogénocarbonate de sodium et l'hydrogénocarbonate de potassium, et la polyvinylpyrrolidone non réticulée comme agent de remplissage.

25. Comprimé selon l'une quelconque des revendications 1 à 24, dans lequel l'adjuvant comprend, sur la base du poids du comprimé nu, 5 à 15% d'adjuvant basique, sélectionné parmi l'hydrogénocarbonate de sodium et l'hydrogénocarbonate de potassium, et 7 à 20% de polyvinylpyrrolidone non réticulée comme agent de remplissage.

26. Comprimé selon l'une quelconque des revendications 1 à 25, dans lequel l'adjuvant se compose d'un adjuvant basique et/ou d'un agent de remplissage neutre à faiblement acide qui améliore la compressibilité.

27. Comprimé selon la revendication 1 ou 2, dans lequel le comprimé nu se compose d'hydrate d'ibuprofène sodique, l'hydrate d'ibuprofène sodique a une teneur en eau de 11 à 16% en poids et la dureté du comprimé est d'au moins 30 N.

28. Comprimé selon la revendication 27, dans lequel l'hydrate d'ibuprofène sodique a une teneur en eau de 12,5 à 15% en poids.

29. Comprimé selon la revendication 27 ou 28, dans lequel la dureté du comprimé est d'au moins 40 N.

30. Comprimé selon l'une quelconque des revendications 1 à 29, dans lequel l'hydrate d'ibuprofène sodique est présent sous forme racémique.

31. Comprimé selon l'une quelconque des revendications 1 à 29, dans lequel l'hydrate d'ibuprofène sodique est présent sous la forme d'hydrate de S(+)-ibuprofène sodique.

32. Comprimé selon l'une quelconque des revendications 1 à 31, dans lequel le comprimé nu est dragéifié ou pelliculé.

33. Comprimé selon l'une quelconque des revendications 1 à 32, dans lequel la couche de sucre ou la pellicule du comprimé nu est présente en une quantité allant de 1 à 10% du poids, sur la base du poids du comprimé nu.

34. Procédé pour produire un comprimé non-effervescent pour administration orale d'ibuprofène sodique, comprenant un comprimé nu et, si on le souhaite, une dragéification ou un pelliculage sur le comprimé nu, dans lequel le comprimé nu se compose de 50 à 100% en poids d'hydrate d'ibuprofène sodique et de 50 à 0% en poids d'adjuvant, sur la base du poids du comprimé nu, et ne contient ni lubrifiant ni agent de délitement, et dans lequel l'hydrate d'ibuprofène sodique a une teneur en eau de 8 à 16% en poids de l'hydrate, ladite teneur étant mesurée comme perte au séchage à 105°C, **caractérisé en ce que** l'hydrate d'ibuprofène sodique, le cas échéant mélangé à l'adjuvant, est compressé en comprimés nus et, si on le souhaite, les comprimés nus sont dragéifiés ou pelliculés.
